(19) Europäisches Patentamt / European Patent Office / Office européen des brevets

(11) **EP 4 714 498 A2**

(12) **DEMANDE DE BREVET EUROPEEN**

(43) Date de publication:
**25.03.2026 Bulletin 2026/13**

(21) Numéro de dépôt: **26157593.0**

(22) Date de dépôt: **21.02.2020**

(51) Classification Internationale des Brevets (IPC):
**A61P 25/28** *(2006.01)*

(52) Classification Coopérative des Brevets (CPC):
**A23L 17/60; A23L 33/105; A23L 33/11; A23L 33/115; A23L 33/12; A61P 25/28**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.02.2019 PCT/FR2019/001820**

(62) Numéro(s) de document de la (des) demande(s) initiale(s) en application de l'article 76 CBE:
**20713704.3 / 3 927 183**

(71) Demandeur: **Microphyt**
**34670 Baillargues (FR)**

(72) Inventeurs:
• **PRADELLES, Remi**
**34670 BAILLARGUES (FR)**
• **DELBRUT, Antoine**
**34670 BAILLARGUES (FR)**

(74) Mandataire: **Cabinet Beau de Loménie**
**103, rue de Grenelle**
**75007 Paris (FR)**

Remarques:
Cette demande a été déposée le 10-02-2026 comme demande divisionnaire de la demande mentionnée sous le code INID 62.

(54) **COMPLÉMENT ALIMENTAIRE**

(57) L'invention concerne un omplément alimentaire comprenant un extrait de *Phaeodactylum tricornutum* comprenant entre 55,1 mg/g et 78,1 mg/g d'extrait d'acides gras de type oméga-3 constitués par ALA, SDA, EPA et DHA, entre 16,0 mg/g d'extrait et 24,0 mg/g d'extrait de fucoxanthine, entre 2,4 mg/g d'extrait et 3,6 mg/g d'extrait de stérols, entre 0,002 mg/g d'extrait et 0,003 mg/g d'extrait de phycoprostanes et de l'huile de coco entre 390 mg/g et 420 mg/g et ses applications en particulier dans la prévention de l'apparition des troubles cognitifs.

**EP 4 714 498 A2**

**Description**

La présente invention concerne une composition, ainsi qu'un complément alimentaire à base d'acides gras et de xanthophylles et leurs applications notamment pour prévenir l'apparition des troubles cognitifs chez l'humain ou l'animal.

Les processus cognitifs sont définis comme l'ensemble des fonctions cérébrales permettant d'acquérir, traiter, mémoriser et utiliser les données issues de l'environnement afin de maximiser les avantages et de minimiser les inconvénients des contraintes extérieures. Ainsi, les processus cognitifs sont à l'œuvre lors des phases impliquant le raisonnement (dont découlent la planification, l'organisation, le jugement), la perception, la reconnaissance, le langage, les émotions, la mémoire et l'apprentissage.

Les troubles légers cognitifs, ou fragilités cognitives, sont définis comme des altérations des fonctions cognitives sans démence. D'un point de vue clinique, ces troubles sont associés à un score de 0,5 dans le cadre d'une évaluation *via* le test CDR (Cognitive Drug Research Computerized Assessment System).

Parmi ces troubles cognitifs, un déclin cognitif survenant avec l'âge et des altérations cognitives induites par un stress prénatal sont deux phénomènes pouvant intervenir au cours de la vie d'un individu.

Le déclin cognitif lié à l'âge est défini comme une diminution non pathologique des fonctions cognitives telles que la vitesse de traitement de l'information, la capacité attentionnelle et surtout la mémoire dite de travail (ou à court terme). Ces processus résultent de modifications physiologiques normales directement corrélées avec l'âge. L'âge du début de ce déclin est encore sujet à controverse, mais étant donné l'accélération du vieillissement de la population mondiale, plus de 20 % de la population mondiale ayant plus de 60 ans, et ce pourcentage sera de plus de 30% en 2050, le déclin cognitif lié à l'âge est un des enjeux majeurs de ces prochaines décennies, au niveau mondial, et spécifiquement au sein des pays développés, dans lesquels il impactera fortement l'économie (moins d'autonomie des personnes âgées) et les politiques publiques.

A l'opposé de la pyramide des âges, des troubles cognitifs peuvent affecter les nourrissons et les jeunes enfants suite à un stress prénatal. En effet, depuis plusieurs années, l'influence du stress durant certaines périodes de la grossesse sur le développement cognitif de l'individu à naitre, a été étudié tant chez l'humain, que chez les animaux. Ainsi, chez les animaux, principalement le rat, il a été montré qu'un stress prénatal chez la mère induisait une descendance possédant une mémoire à long terme altérée.

Il apparait que des stimuli négatifs intenses, des stress, peuvent induire des altérations ou diminutions non pathologiques des fonctions cognitives des jeunes enfants, se manifestant par une hyperactivité, un déficit d'attention et de mémoire, des retards au niveau du langage, des tempéraments plus difficiles, et plus globalement des altérations comportementales telles qu'un comportement anxieux, traduisant un retard au point de vue neurodéveloppemental et une baisse des capacités cognitives.

Un des mécanismes supposés de la traduction du stress prénatal en troubles cognitifs, est basé sur la mise en contact du fœtus avec des doses importantes d'hormones, dites du stress, appartenant à la famille des corticostéroïdes, telles que le cortisol. Or, le cortisol passe la barrière placentaire et, à partir d'une certaine concentration, les mécanismes de protection du fœtus contre les corticoïdes sécrétés par la mère, sont saturés, mettant ainsi le fœtus en contact avec des doses trop importantes de cortisol qui semblent avoir un effet délétère sur le développement cognitif. D'autres hypothèses complémentaires expliquant le lien entre stress prénatal et troubles cognitifs de l'enfant, sont avancées.

La notion de stress peut être définie selon différents angles tels que l'approche biologique : le stress est alors une série de réactions métaboliques, suite à un ou des facteurs exogènes, induisant des changements physiologiques ou psychologiques (peur, angoisse) au sein de l'organisme. Cependant, la notion de stress et ses impacts est largement individu-dépendante, et la réponse de l'individu à un stress se définit aussi sous l'angle psychologique. Dès lors, étant donné qu'un évènement n'est stressant qu'a *posteriori,* du fait d'une réaction propre à chaque individu, ou qu'il peut être objectivement stressant, il est difficile d'agir sur les sources du stress prénatal. A ceci s'ajoute le fait que la grossesse induit des changements hormonaux et psychologiques augmentant la sensibilité de la future mère à tout évènement pouvant avoir un lien avec le bien-être de l'enfant à naitre.

L'approche strictement thérapeutique du stress ou de l'anxiété, par voie médicamenteuse, est périlleuse dans le cas de la femme enceinte : de nombreux psychotropes pour le traitement des troubles psychologiques ou de l'anxiété ont des effets tératogéniques avec des conséquences directes néfastes sur le fœtus. Ceci nécessite une évaluation au cas par cas, et cette approche n'est utilisée que dans le cas de troubles psychologiques cliniques chez la femme enceinte et non dans le cas de stress dits subjectifs.

Ainsi, il existe une problématique forte portant sur la découverte de solutions contre les conséquences du stress prénatal sur les troubles cognitifs de l'enfant ou du jeune adulte.

Différentes études ont montré l'intérêt d'une supplémentation nutritionnelle en acides gras dits essentiels, mais aussi en carotènes et notamment en xanthophylles, voire en l'association desdits acides gras et desdits carotènes, pour prévenir ou au moins limiter le déclin des fonctions cognitives. Des compléments alimentaires ou des médicaments ont été développés conduisant à des résultats encourageants.

Ainsi on connait selon le document WO2013/032333A1, une composition à base d'acides gras de type oméga-3, en particulier l'acide éïcosapentaénoïque (EPA) et l'acide docosahexaénoïque (DHA), d'asthaxanthine et de glycérophospholipides, qui est préconisée pour la prévention ou le traitement de différents troubles et notamment des troubles cognitifs. Lesdits ingrédients sont présents dans cette composition sous forme d'extraits de microalgues ; dans une variante préférée de préparation, ils sont obtenus par formulation de deux extraits provenant de deux algues différentes. L'origine naturelle des constituants de la composition est un réel atout. Toutefois, le besoin de compositions plus efficaces existe, en particulier eu égard aux enjeux cités précédemment. Il est en outre important de disposer de procédés de préparation de telles compositions simples et reproductibles.

L'invention apporte une solution avec une composition comprenant un ou des acides gras de type oméga-3 et un ou des xanthophylles, ainsi qu'un ou des composés de la famille des stérols et un ou des phycoprostanes. Il s'est avéré que l'association d'au moins un stérol et d'au moins un phycoprostane avec au moins un acide gras de type oméga-3 et au moins un xanthophylle augmente significativement l'efficacité d'une composition en prévention l'apparition des troubles cognitifs liés à l'âge mais aussi contre ceux associés à un stress prénatal.

Une composition de l'invention comprend :

au moins 50 mg/g d'un ou plusieurs acides gras de type oméga-3,
au moins 10 mg/g d'un ou plusieurs xanthophylles,
au moins 1 mg/g d'un ou plusieurs stérols et
au moins 2 $\mu$g/g d'un ou plusieurs phycoprostanes.

Dans une indication principale, une composition de l'invention peut être utilisée comme complément alimentaire. Aussi l'invention concerne un complément alimentaire qui comprend au moins 50 mg/g d'un ou plusieurs acides gras de type oméga-3, au moins 10 mg/g d'un ou plusieurs xanthophylles, au moins 1 mg/g d'un ou plusieurs stérols et au moins 2 $\mu$g/g d'un ou plusieurs phycoprostanes.

L'invention présente un avantage essentiel dans le fait que l'ensemble des constituants ou ingrédients ci-dessus peuvent être obtenus à partir d'une source naturelle et en particulier peuvent être extraits d'une ou de plusieurs microalgues, et de préférence d'une seule microalgue. Bien entendu, l'un ou plusieurs des constituants ou ingrédients d'une composition ou d'un complément alimentaire de l'invention peuvent être d'origine non naturelle et apportés sous forme de produits fabriqués par synthèse chimique.

Avant d'exposer l'invention plus en détails, certains termes employés dans le présent texte sont définis.

Le terme « comprend » dans l'expression « une composition comprend » ou « un complément alimentaire comprend » signifie que la composition ou le complément peut incorporer un quelconque constituant supplémentaire ou plus, non expressément mentionné, sous quelle que forme et de quelle qu'origine que ce soit. Il couvre aussi une composition ou un complément qui ne contiendrait que les constituants listés et qu'en conséquence la composition ou le complément consisterait en lesdits constituants.

Un complément alimentaire est défini comme une ou des denrées alimentaires dont le but est de compléter le régime alimentaire normal d'un humain ou d'un animal, et qui constituent une source concentrée de nutriments ou d'autres substances ayant un effet nutritionnel ou physiologique seuls ou combinés ; il est généralement disponible sous forme de doses, à savoir les formes de présentation telles que les gélules, les pastilles, les comprimés, les pilules et autres formes similaires, ainsi que les sachets de poudre, les ampoules de liquide, les flacons munis d'un compte-gouttes et les autres formes analogues de préparations liquides ou en poudre destinées à être prises en unités mesurées de faible quantité.

Le ou les acides gras de type oméga-3 sont une famille d'acides gras insaturés dont la chaîne hydrocarbonée a de l'ordre de 4 à 36 atomes de carbone, généralement de l'ordre de 14 à 36 atomes de carbone, et dont la double liaison ou la première double liaison, comptée depuis le groupe méthyle terminal de la chaîne, se trouve sur la troisième liaison carbone-carbone. La ou les insaturations peuvent être indépendamment les unes des autres, cis ou trans. Les acides les plus représentatifs sont l'acide alpha-linolénique (ALA), l'acide éïcosapentaénoïque (EPA) et l'acide docosahexaénoïque (DHA), mais l'appellation « acides gras de type oméga-3 » n'y est pas restreinte. En outre, et en particulier quand le ou les acides gras sont d'origine naturelle, ils peuvent être extraits d'algues et se présenter sous la forme de molécules libres mais aussi sous une forme dérivée telle qu'une forme estérifiée, par exemple sous forme mono-, di- ou tri-estérifiée, ou en mélanges de ces formes.

Par xanthophylles, sont définis les molécules appartenant aux caroténoïdes comportant un ou plusieurs atomes d'oxygène tels que l'astaxanthine, la cantaxanthine, la vaucheriaxanthine, la lutéine, la zéaxanthine, la diadinoxanthine, la néoxanthine, la loroxanthine, la siphonoxanthine, la diatoxanthine, la violaxanthine, la dinoxanthine, la flavoxanthine, l'$\alpha$-cryptoxanthine, la $\beta$-cryptoxanthine et la fucoxanthine. Notamment quand le ou les xanthophylles sont d'origine naturelle, ils peuvent être extraits d'algues et se présenter sous la forme de molécules libres mais aussi sous une forme dérivée telle qu'une forme estérifiée de mono- ou pluri-esters, ou en mélanges de ces formes.

Les stérols sont une famille de lipides bien connus possédant un noyau de stérane dont le carbone en position 3 est porteur d'un groupe hydroxyle, ce dernier pouvant être modifié par exemple par un groupe acétyle. Ils incluent les stérols naturels ou phytostérols, et sont regroupés dans le présent texte sous le terme de phycostérols. De façon non exhaustive, on peut citer à titre de phytostérols, le 24-méthylène-cholestérol, le β-sitostérol, le fucostérol, l'isofucostérol, le saringostérol, l'oxocholestérol acétate, le crinostérol, et plus particulièrement le brassicastérol, le stigmastérol et le campestérol.

Par phycoprostane, on entend une famille de lipides structurellement de type prostaglandine, d'origine naturelle, résultant d'oxydations non directement enzymatiques des acides gras présents naturellement au sein des biomasses microalgales. Ces composés sont en particulier choisis parmi les phytoprostanes, les isoprostanes et les neuroprostanes, selon l'acide gras qui a subi la ou des oxydations. Ainsi, ces composés peuvent être issus des acides gras tels que l'acide α-linolénique (ALA), l'acide arachidonique (ARA), l'acide éïcosapentaénoïque (EPA), ou l'acide docosahexaénoïque (DHA). Les phytoprostanes sont principalement dérivés de l'ALA et peuvent être choisis parmi le 9-epi-9F1t-PhytoP, le ent-16-epi-16-F1t-PhytoP, le 9-F1t-PhytoP, le ent-16B1t-PhytoP, le ent-9L1t-PhytoP, le 16(RS)-16-A1t-PhytoP. Les isoprostanes sont principalement dérivés de l'ARA et de l'EPA et peuvent être choisis parmi le 15-E2t-IsoP, le 15-F2t-IsoP, le 15-epi-15-F2t-IsoP, le 5-F2t-IsoP, le 8(RS)-8-F3t-IsoP. Les neuroprostanes sont principalement dérivés du DHA et peuvent être choisis parmi le 4-F3t-NeuroP, le 10-F4t-NeuroP, le 10-epi-10-F4t-NeuroP, le 4(RS)-4-F4t-NeuroP, le 14(RS)-14-F4t-NeuroP, le 20(R)-20-F4t-NeuroP.

Par triglycérides à chaines moyennes (MCT, Medium Chain Triglycerides), on comprend des esters de glycérol et d'acides gras saturés, dont la chaîne hydrocarbonée a de l'ordre de 6 à 12 atomes de carbone. Ils sont naturellement présents dans l'huile de coco telle que l'huile de noix de coco, l'huile de palmiste et l'huile de palme, mais peuvent être obtenus à partir d'autres graisses ou huiles.

La présente invention est ci-après décrite plus en détails et ses variantes exposées.

Une composition ou un complément alimentaire de l'invention répond avantageusement aux caractéristiques suivantes, considérées seules ou en une ou plusieurs quelconques combinaisons.

Elle ou il comprend 50 à 250 mg/g d'un ou plusieurs acides gras de type oméga-3, 10 à 50 mg/g d'un ou plusieurs xanthophylles, 1 à 20 mg/g d'un ou plusieurs stérols et 2 à 100 $\mu$g/g d'un ou plusieurs phycoprostanes.

Elle ou il comprend 50 à 200 mg/g d'un ou plusieurs acides gras de type oméga-3, 10 à 30 mg/g d'un ou plusieurs xanthophylles, 1 à 8 mg/g d'un ou plusieurs stérols et 2 à 50 $\mu$g/g d'un ou plusieurs phycoprostanes.

Elle ou il comprend 50 à 170 mg/g d'un ou plusieurs acides gras de type oméga-3, 10 à 25 mg/g d'un ou plusieurs xanthophylles, 1 à 6 mg/g d'un ou plusieurs stérols et 2 à 40 $\mu$g/g d'un ou plusieurs phycoprostanes.

Avantageusement, une composition ou un complément alimentaire de l'invention contient en outre et à titre de véhicule ou support pour faciliter l'expression des ingrédients actifs, au moins une huile. De manière surprenante, il a été observé que la fabrication d'une composition ou d'un complément alimentaire sont facilitées lorsque cette huile est choisie parmi les triglycérides à chaines moyennes (MCT). En particulier, lorsque les ingrédients actifs sont obtenus à partir d'un même extrait de microalgue, une homogénéisation optimale est constatée dans une telle huile. Selon une variante, les triglycérides à chaines moyennes (MCT) sont d'origine naturelle et sont apportés par une huile choisie parmi l'huile de coco, l'huile de palmiste et l'huile de palme ; ils peuvent aussi être obtenus ou dérivés d'une telle huile.

Ci-après, des formulations préférées d'une composition ou d'un complément alimentaire de l'invention sont présentées, ces mises en œuvre pouvant bien entendu être combinées :

le ou au moins l'un des acides gras de type oméga-3 est choisi parmi l'acide stéaridonique (SDA), l'acide éicosapentaénoïque (EPA), l'acide docosahexaénoïque (DHA) et leurs mélanges ;
le ou au moins l'un des xanthophylles est la fucoxanthine ;
le ou au moins l'un des stérols est choisi parmi les phytostérols ;
le ou au moins l'un des phycoprostanes est choisi parmi les phytoprostanes, les isoprostanes et les neuroprostanes.

Une composition ou un complément alimentaire de l'invention peut comprendre tout additif permettant notamment d'en améliorer la conservation, l'aspect, le goût, la formulation. Ainsi, un ou plusieurs additifs tels que ceux choisis parmi les agents conservateurs, les colorants, les arômes, les agents de délitement, les agents lubrifiants, les agents d'enrobage ou d'encapsulation peuvent y être incorporés.

Une application majeure d'une composition de l'invention est nutraceutique, ainsi une telle composition ou un complément alimentaire tel que défini ci-dessus se présente avantageusement sous la forme de gélules, de capsules, de comprimés, de pastilles ou de poudre libre. Elle ou il est de préférence conditionné en doses ayant un poids unitaire compris entre 1 mg et 1 g. De manière générale, la galénique de la composition ou du complément sera adaptée au sujet considéré et en particulier selon qu'elle est destinée à un enfant ou un adulte.

Une composition ou un complément alimentaire de l'invention peut être utilisée pour prévenir l'apparition des troubles cognitifs non pathologiques liés au vieillissement ou les troubles cognitifs non pathologiques chez les enfants ou les jeunes adultes ayant subi un stress prénatal. Dans la prévention des troubles cognitifs liés au vieillissement, la prise journalière pourra être comprise entre 2 à 5 mg/kg de poids corporel. Dans la prévention des troubles cognitifs chez les enfants ou les jeunes adultes ayant subi un stress prénatal, la prise journalière pourra être comprise entre 0,05 à 0,1 mg/kg de poids corporel. Il a été remarqué qu'un effet est observé même pour de très faibles prises journalières, dès lors que la durée du traitement est proportionnellement rallongée.

L'invention concerne aussi l'utilisation d'une microalgue pour préparer un complément alimentaire tel que défini précédemment. Une ou des microalgues préférées sont choisies dans l'un quelconque des taxons suivants *Pinguiophyceae, Chrysophyceae, Bacillariophyceae, Mamiellophyceae, Prymnesiophyceae, Haptophyceae, Coccolithophyceae, Isochrysidaceae* et *Phaeodactylaceae.* Avantageusement la microalgue est la *Tisochrysis lutea* ou la *Phaeodactylum tricornutum.* On optera pour de telles microalgues car une extraction appropriée conduit à un extrait dont la composition répond à la définition d'une composition de l'invention. A titre d'exemple, un tel extrait peut comprendre la fraction d'acides gras suivante : les acides gras, exprimés en pourcentage massique d'extrait total, sont sous forme d'acides gras libres entre 4 et 55 %, sous forme de monoacyl glycérol entre 0,5 et 10 %, sous forme de diacylglycérol entre 0,4 et 15 % et sous forme de triacyl glycérol entre 2 et 55%. Ces acides gras sont à hauteur de 5 à 20% (m/m) des acides gras de la série oméga-3 et entre 0,5 et 5 %, des acides gras de la série oméga-6. Plus précisément, les acides gras sont notamment de l'ALA (acide $\alpha$-linolénique) entre 0,5 et 10%, du SDA (acide stéaridonique) entre 0,5 et 10%, de l'EPA (acide eicosapentaénoïque) entre 0,05 et 20%, du DHA (acide docosahexaénoïque) entre 0,1 et 10%.

Comme indiqué précédemment, un des intérêts d'une composition ou d'un complément alimentaire réside dans son procédé de préparation et précisément l'origine naturelle de ses constituants qui peuvent tous être obtenus à partir d'une seule et même microalgue. Selon la microalgue utilisée, la formulation de la composition peut être directement obtenue avec l'extrait. Si tel n'est pas le cas, l'extrait sera dilué pour obtenir les concentrations requises selon l'invention. L'invention n'est toutefois pas limitée à cette mise en œuvre, ainsi il peut être envisagé que seulement une partie des constituants soit d'origine naturelle, les autres étant obtenus par voie de synthèse chimique et/ou que les constituants d'origine naturelle ne soient pas issus de la même source, par exemple ne soient pas produits à partir de la même algue.

La mesure et l'ajustement des concentrations des ingrédients actifs dans un extrait, et dans la composition ou le complément alimentaire obtenu, sont réalisées à l'aide de techniques analytiques qui appartiennent aux connaissances générales de l'homme du métier.

Un procédé de fabrication d'une composition ou d'un complément alimentaire à partir d'une culture de microalgue est ci-après décrit plus en détails.

Selon une variante de l'invention, lesdits organismes sont des microalgues, telles que celles appartenant aux taxons *Pinguiophyceae, Chrysophyceae, Bacillariophyceae, Mamiellophyceae, Prymnesiophyceae, Haptophyceae, Coccolithophyceae, Isochrysidaceae, Phaeodactylaceae.* Ces microorganismes capables de photosynthèses peuvent être autotrophes stricts, mixotrophes ou hétérotrophes transitoirement. Ces organismes peuvent être récoltés dans un environnement naturel ou préférentiellement cultivés.

Par extrait, on désigne une fraction de la biomasse issue des organismes capables de photosynthèse obtenus par un procédé permettant d'obtenir, directement ou indirectement, une composition de l'invention. Ces extraits ont une composition, exprimée en pourcentage massique de l'extrait total, en protéines comprises entre 5 et 30%, en lipides entre 20 et 80%, entre 0,1 et 2% de stérols, entre 0,1 et 20% de chlorophylle.

Plus précisément, la partie lipophile composant l'extrait, exprimée en pourcentage massique d'extrait total, est constitué d'acides gras saturés entre 15 et 45%, d'acides gras polyinsaturés entre 5 et 20%, de xanthophylles entre 1 et 20% et de phycoprostane entre 0,0002 et 0,007%.

Aux fins de la production de l'extrait selon l'invention, avantageusement les cellules sont des cellules de microalgues de l'espèce *Tisochrysis lutea* de la famille des *Isochrysidaceae,* ou des cellules de microalgues de l'espèce *Phaedactylum tricornutum* de la famille des *Phaeodactylaceae,* produites par autotrophie vis-à-vis du carbone.

Mode de production des microalgues

Les microalgues sont idéalement cultivées de façon contrôlée au sein de systèmes adaptés tels que des raceways, open ponds ou préférentiellement des systèmes fermés de type photobioréacteurs. Les photobioréacteurs utilisés peuvent être de tout type existants tels que des photobioréacteurs tubulaires horizontaux, verticaux tels que des systèmes dits « green wall panel », des photobioréacteurs plans ou en colonne. Préférentiellement, la production de la biomasse s'effectuera au sein d'un système de culture fermé, par autotrophie sans impact sur les terres arables.

La production de la biomasse s'effectue selon les modes de conduite de culture de type batch, fed-batch, continu, semi-continu, turbidostat ou chemostat.

Obtention des extraits de ces microalgues

Les extraits issus de ces microorganismes sont obtenus préférentiellement après concentration de la biomasse par élimination de tout ou partie de l'eau en utilisant des procédés chimiques ou physiques tels que la centrifugation, la filtration, la floculation, la sédimentation, couplés, ou non, à des étapes de séchage par lyophilisation, séchage sous vide, séchage tambour, atomisation ou tout autre procédé permettant de baisser la teneur en eau de la biomasse. En complément à ces étapes, des procédés de lyses cellulaires peuvent être mis en œuvre tels que l'applications de pressions, de flux électriques de forces de cisaillements, l'utilisation d'enzymes, ou tout autres procédés permettant la destructuration des tissus, organes, cellules ou organites.

Les composés d'intérêt de la biomasse sont extraits selon un procédé de type extraction solide-liquide, pouvant être par fluides hypercritiques ou par fluides subcritiques, pouvant faire intervenir des co-traitements menés en parallèle ou en séquentiel de types micro-ondes, ultrasons, pressions, enzymatiques. Les solvants utilisés purs ou en mélange peuvent être de l'acétone, de l'hexane, de l'acétate d'éthyle, du méthyltétrahydrofurane, de l'heptane, du méthanol, des huiles naturelles ou ramifiées, de l'éthanol ou tout autre solvant permettant d'extraire tout ou partie des composés de natures hydrophobes et amphiphiles.

Le solvant ou le mélange de solvants est séparé de la biomasse résiduelle après extraction par des procédés de type centrifugation, filtration et peut par la suite être concentré, ou le solvant éliminé, par des techniques telles que l'évaporation sous vide ou tout autre technique permettant l'évaporation sélective du solvant considéré. L'extrait ainsi obtenu est de nature lipophile tout en comportant des molécules amphiphiles.

Formulation en tant que complément alimentaire

La formulation de l'extrait s'effectue avec des matrices compatibles, permettant sa dissolution afin d'obtenir une solution homogène de concentration voulue en extrait, comme par exemple, des huiles végétales telles que l'huile d'olive, l'huile de colza, l'huile de lin, l'huile de tournesol, l'huile de pépins de raisins, l'huile de palme et préférentiellement les huiles MCT, et composées à plus de 70% en masse d'un mélange d'acide caprylique et d'acide caprique, et de préférence choisies parmi l'huile de noix de coco ou l'huile de palme, le tout complémenté avec des molécules permettant d'augmenter la stabilité tels que des anti-oxydants de synthèse ou naturels. Les taux massiques d'incorporation des matrices/des additifs afin d'obtenir le complément peuvent atteindre 95% en poids par rapport au poids du complément alimentaire, ils sont généralement compris entre 15 et 80 %, préférentiellement entre 35 et 45%.

L'extrait, mais préférentiellement la composition formulée ou le complément obtenu, peuvent être mis sous forme de capsules molles, ou peuvent être formulés sous forme de poudre, par toute technique permettant la microencapsulation de solution aqueuse faisant intervenir ou non un support ou une matrice permettant ou non sa dispersibilité homogène au sein d'une solution buvable polaire.

L'extrait ou le complément peut être utilisé seul ou comme ingrédient au sein d'une complémentation alimentaire.

Les différents objets de l'invention sont ci-dessous illustrés et leurs avantages mis en évidence dans les exemples suivants, à l'appui des figures suivantes :

[Fig. 1] est une représentation des effets du complément de l'invention sur l'activité locomotrice, avec le diagramme de gauche illustrant les effets sur les déficits d'alternances spontanées et le diagramme de droite illustrant les effets sur l'activité locomotrice.

[Fig. 2] est une représentation des effets sur les déficits d'apprentissage induits par le D-Gal selon le test MWM.

[Fig. 3] est une représentation des effets du complément et du DHA sur les déficits d'apprentissage induits par le D-Galactose.

[Fig. 4] est une représentation des effets sur les déficits d'évitements passifs induits par le D-Galactose chez la souris, avec les effets sur la latence de passage à pas illustrés sur le diagramme de gauche et sur la latence d'échappement illustrés sur le diagramme de droite, mesurés pendant la période de rétention.

[Fig. 5] est une représentation des effets du complément et du DHA sur la peroxydation lipidique induite par le D-Galactose

[Fig. 6] est une représentation des effets du complément et du DHA sur l'expression induite de TNF-$\alpha$ dans le cortex et le plasma par le D-Galactose, avec l'effet sur le cortex sur le diagramme de gauche et l'effet sur le plasma sur le diagramme de droite.

[Fig. 7] est une représentation des effets du complément et du DHA sur l'expression induite par le D-Galactose, d'IL-6 dans le cortex (diagramme de gauche) et le plasma (diagramme de droite).

[Fig. 8] est une représentation de l'effet du complément sur l'anxiété, dans le test de locomotion au centre de l'espace de test, jour JPN46.

[Fig. 9] est une représentation de l'effet du complément sur la mémoire de reconnaissance, dans le test de reconnaissance d'un objet, jour JPN47.

[Fig. 10] est une représentation de l'effet du complément sur la mémoire de reconnaissance, dans le test de reconnaissance d'un nouvel objet.

[Fig. 11] est une représentation des effets du complément de l'invention sur l'activité locomotrice, avec le diagramme de gauche illustrant les effets sur les déficits d'alternances spontanées et le diagramme de droite illustrant les effets sur l'activité locomotrice.

[Fig. 12] est une représentation des effets sur les déficits d'apprentissage induits par le D-Gal selon le test MWM.

[Fig. 13] est une représentation des effets du complément sur les déficits d'apprentissage induits par le D-Galactose.

[Fig. 14] est une représentation des effets sur les déficits d'évitements passifs induits par le D-Galactose chez la souris, avec les effets sur la latence de passage à pas illustrés sur le diagramme de gauche et sur la latence d'échappement illustrés sur le diagramme de droite, mesurés pendant la période de rétention.

[Fig. 15] est une représentation des effets du complément sur la peroxydation lipidique induite par le D-Galactose

[Fig. 16] est une représentation des effets du complément sur l'expression induite de TNF-$\alpha$ dans le cortex et le plasma par le D-Galactose, avec l'effet sur le cortex sur le diagramme de gauche et l'effet sur le plasma sur le diagramme de droite.

[Fig. 17] est une représentation des effets du complément sur l'expression induite par le D-Galactose, d'IL-6 dans le cortex (diagramme de gauche) et le plasma (diagramme de droite).

## Exemple 1: Formulation d'un extrait contenant les constituants d'une composition de l'invention.

Un extrait est obtenu selon l'une des techniques décrites ci-dessus à partir de la microalgue *Phaeodactylum tricornutum.*

Il est insoluble dans l'eau et est hautement visqueux empêchant toute manipulation à température ambiante.

L'extrait et l'huile de palme sont mis à température ambiante (25 ± 1 °C) 24 h avant la préparation. L'extrait est transféré dans un tube à centrifuger comportant l'huile de telle manière que la masse nette finale du mélange soit d'environ 5 g et la proportion massique soit telle que l'extrait consiste en 25 % de la masse totale nette du mélange. Le mélange est agité pendant une minute par utilisation d'un dispositif de mélange dit vortex. L'agitation est répétée trois fois par mélange. On obtient un mélange homogène.

## Exemple 2 : Test d'un extrait naturel de la microalgue *Tisochrysis lutea* dans le cadre du modèle *in vivo* sur l'atténuation des déficits induits par le déclin cognitif lié à l'âge

Le complément alimentaire de l'invention est préparé à partir d'un extrait de *Tisochrysis lutea* qui comprend en mg/g :

Acides gras de type oméga-3 (ALA, SDA, EPA, DHA) : 152,6 ± 14,4 ;
Fucoxanthine : 20,0 ± 4,0 ;
Stérols : 4,9 ± 0,8 ;
Phycoprostane : 0,035 ± 0,007

Le complément est obtenu par addition d'huile de noix de coco à raison de 360 mg ± 10 mg/g audit extrait.

Le complément est incorporé à des croquettes selon 3 formulations différentes telles que les concentrations finales en DHA de ces lots de croquettes soient égales à 0,5, 1,5 et 3,0 % (m:m).

Un extrait huileux commercial de microalgues ne comprenant à titre de fraction grasse que les acides gras DHA 77 % (m:m) et EPA 3% (m:m) est également testé ; il est aussi incorporé à un lot de croquettes tel que la concentration finale en DHA de ce lot de croquettes soit égale à 3,1% (m:m).

Un lot supplémentaire de croquettes est formulé uniquement avec l'huile de noix de coco, tel que la concentration en véhicule soit équivalente à celle des autres lots, soit 0,01 % (m:m).

Les cinq lots de croquettes ainsi obtenus sont référencés tels que décrit dans le tableau 1 ci-dessous.

[Table 1]

| Croquettes formulées | Référence | [DHA] en % (m:m) |
|---|---|---|
| Huile de noix de coco | A1 | 0 |
| Complément | A2 | 0,5 |
| Complément | A3 | 1,5 |
| Complément | A4 | 3,0 |
| Extrait huileux commercial | A5 | 3,1 |

Le modèle *in vivo* considéré est le modèle D-Galactose appliqué à des souris qui est adapté à l'étude du déclin cognitif lié à l'âge. Ce modèle imite en effet de nombreuses caractéristiques comportementales et moléculaires du vieillissement cérébral dans les modèles de rongeurs.

Le D-Galactose est administré par voie sous-cutanée à raison de 150 mg/kg de poids frais de souris par jour, et le complément alimentaire ci-dessus est incorporé dans un granulé, selon le schéma suivant :

- Entre le jour -14 et le jour 51, le complément est administré par incorporation dans des granulés alimentaires ;
- Entre le jour 01 et le jour 51, le D-Galactose est administré par voie sous-cutanée, cinq jours par semaine ;
- Entre les jours 43 et 51, trois tests comportementaux différents sont utilisés pour surveiller les effets des composés d'essai.

L'efficacité du complément est évaluée selon les paramètres suivants : amélioration des déficits d'apprentissage (mémoire de travail spatiale : alternance spontanée dans le labyrinthe en Y selon le test Y-maze ; mémoire spatiale par le test dit « Morris Water Maze » et mémoire contextuelle à long terme dans le test d'évitement passif), taux de peroxydation lipidique (LPO) dans l'hippocampe et l'effet sur les marqueurs de neuro-inflammation IL6 et TNF$\alpha$.

Amélioration des déficits d'apprentissage

- Au jour 43, tous les animaux ont été testés pour la performance d'alternance spontanée dans le test Y-maze (YM), via un indice de mémoire de travail spatiale ;
- Du jour 44 jusqu'au jour 49, tous les animaux ont été testés pour la mémoire spatiale dans le test Morris Water Maze (MWM), via un indice de mémoire spatiale ;
- Du jour 44 jusqu'au jour 49, tous les animaux sont testés via le test MWM pour évaluer la mémoire de travail spatiale ;
- Les jours 50 et 51, la mémoire contextuelle à long terme des animaux est évaluée à l'aide de la procédure d'évitement passif de type pas-à-pas (STPA), via des séances d'entraînement et de rétention, respectivement ;
- Les jours 50 et 51, tous les animaux sont testés pour la tâche STPA.

Taux de peroxydation lipidique (LPO) dans l'hippocampe et effet sur les marqueurs de neuro-inflammation IL6 et TNF$\alpha$

Le 51e jour, après les tests comportementaux, les animaux sont euthanasiés.

Pour tous les animaux, le sang du tronc est prélevé et centrifugé pour récupérer le plasma, et le cerveau est rapidement prélevé. L'hippocampe et le cortex sont disséqués, l'hippocampe est ensuite utilisé pour déterminer les taux de peroxydation lipidique par méthode colorimétrique ; le cortex hémi-frontal et le plasma sont utilisés pour déterminer le niveau des biomarqueurs inflammatoires interleukine-6 (IL-6) et le facteur alpha de nécrose tumorale (TNF-$\alpha$).

La quantification des taux de peroxydation lipidique (LPO) a été réalisée selon la procédure modifiée et adaptée de Hermes-Lima et col.. Cette méthode mesure la capacité des lipides peroxydés du cerveau à oxyder un complexe d'oxyde ferreux et de xylénol orange, mise en évidence en présence d'hydroperoxyde de cumère (CHP). Le niveau de peroxydation lipidique est déterminé en équivalent-CHP selon :

$$CHPE = A5801/A5802 \times [CHP\ (nmol)]$$ et exprimé en équivalent-CHP par poids humide de tissu et en pourcentage comparativement aux données obtenues pour le groupe témoin (D-Galactose + véhicule).

Les teneurs en IL6 et TNF$\alpha$ sont quantifiées au moyen de tests ELISA avec les kits suivants :

Pour la quantification de l'IL6 : ThermoScientifique, EM2IL6
Pour la quantification du TNF$\alpha$ : ThermoScientifique, EMTNFA

Pour tous les essais, le cortex est homogénéisé après décongélation dans un tampon de 50 mM Tris-150 mM NaCl, pH 7,5, et soniqué pendant 20 s. Après centrifugation (16 100 g pendant 15 min, 4 °C), un surnageant ou du plasma sont utilisés pour les essais ELISA conformément aux instructions du fabricant des tests ELISA. Pour chaque essai, l'absorbance est lue à 450 nm et la concentration de l'échantillon calculée à l'aide de la courbe étalon. Les résultats sont exprimés en pg de marqueur par mg de tissu frais.

Toutes les valeurs, à l'exception des latences d'évitement passif, sont exprimées en moyenne plus ou moins l'écart type de mesure. Des analyses statistiques sont effectuées séparément pour chaque composé à l'aide d'une ANOVA unidirectionnelle (valeur F), suivies du test de comparaison multiple post-hoc de Dunnett. Les latences d'évitement passives ne suivent pas une distribution gaussienne, puisque les temps limites supérieurs sont fixés. Elles sont donc analysées à l'aide d'une ANOVA non paramétrique Kruskal-Wallis (valeur H), suivie d'un test de comparaison multiple de Dunn. Les valeurs avec $p < 0,05$ sont considérées comme statistiquement significatives.

Les tests sont effectués sur 60 souris mâles, réparties dans 6 groupes de 10 souris, parmi lesquels le groupe 1 est le groupe témoin négatif et les groupes 2-6 sont les groupes témoins positifs :

le groupe 1 est le groupe auquel est administré une solution saline sous cutanée au lieu du D-Galactose et des croquettes A1 ;

le groupe 2 est le groupe auquel sont administrés du D-Galactose et des croquettes A1 ;

le groupe 3 est le groupe auquel sont administrés du D-Galactose et des croquettes A2 ;

le groupe 4 est le groupe auquel sont administrés du D-Galactose et des croquettes A3 ; et

le groupe 5 est le groupe auquel sont administrés du D-Galactose et des croquettes A4 ; et

le groupe 6 est le groupe auquel sont administré du D-Galactose et des croquettes A5.

Le calcul de la dose journalière équivalente chez l'humain, à partir de la dose journalière testée chez la souris est défini comme suit par la FDA (Guidance, 2005) : la dose journalière chez l'humain exprimée en mg/kg de poids corporel (HED Human) est égale à la dose journalière chez l'animal exprimée en mg/kg de poids corporel (HED Animal) multipliée par le ratio du facteur de sécurité (Km Animal) chez l'animal considéré et du facteur de sécurité pour l'homme (Km Humain). Le Km Humain est égal à 37 et le Km Souris est égal à 3.

Effets sur la mémoire spatiale dans l'alternance spontanée du test Y-maze :

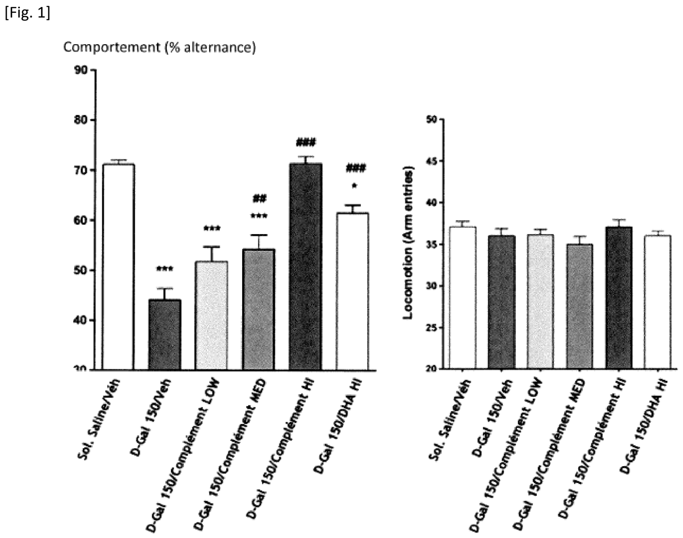

Les résultats sont représentés à la figure 1, le premier diagramme (à gauche) illustrant les effets du complément de l'invention sur les déficits d'alternances spontanées et le second diagramme (à droite) illustrant les effets du complément de l'invention sur l'activité locomotrice.

Sur la figure 1 : LOW, faible dose du complément (A2) ; MED, dose moyenne du complément (A3) ; HI, dose élevée du complément (A4) ; N est compris entre 9 et 10 selon les groupes ; * $p < 0,05$, *** $p < 0,0001$ vs. la solution saline / groupe Veh, # $p < 0,05$, ## $p < 0,01$, ### $p < 0,0001$ vs. le groupe D-GAL 150 / groupe Veh; test de Dunnett.

On observe que le traitement au D-Galactose a significativement altéré la mémoire de travail spatiale, comparativement aux souris traitées par solution saline.

Le complément A2 n'a montré aucun effet sur le comportement d'alternance. Le complément A3 a très significativement mais partiellement atténué les déficits induits par l'intoxication chronique au D-Galactose. Le complément A4 a très significativement et complètement atténué les déficits induits par l'intoxication chronique au D-Galactose.

Le traitement au DHA seul (selon A5) a très significativement mais partiellement allégé les déficits induits par l'intoxication chronique au D-Gal.

De manière étonnante, il apparait que le traitement préventif par le complément de l'invention a un effet positif supérieur (très significatif et atténuation complète des déficits) comparativement au traitement au DHA seul (très significatif et atténuation partielle des déficits), et ce pour une même dose de DHA.

Effets sur les déficits d'apprentissage induits par le D-Gal selon le test MWM :

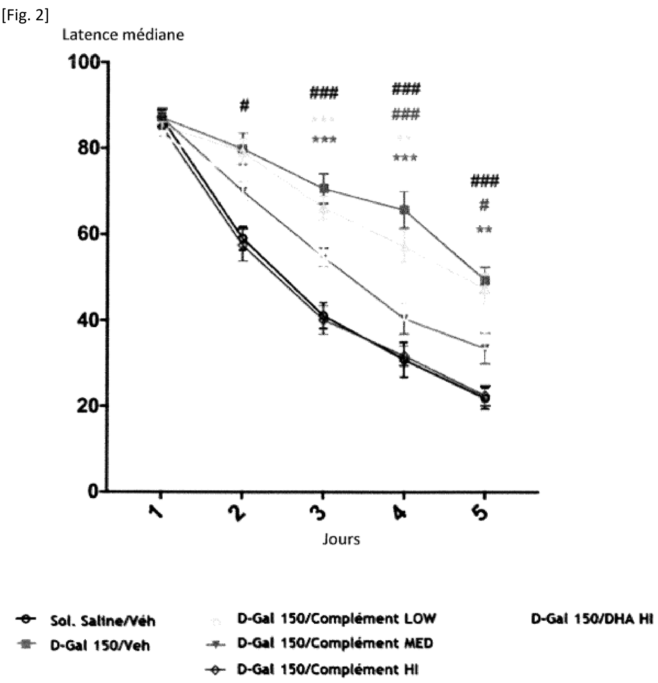

Les résultats sont représentés à la figure 2.

Sur la Figure 2 : LOW, faible dose du complément (A2) ; MED, dose moyenne du complément (A3) ; HI, dose élevée du complément (A4) ; N est compris entre 9 et 10 selon les groupes ; * $p < 0,05$, ** $p < 0,01$, *** $p < 0,0001$ vs. solution saline / groupe Veh, ## $p < 0,01$, ### $p < 0,0001$ vs. groupe D-GAL 150 / groupe Veh; le test de comparaison multiple de Bonferroni après ANOVA bidirectionnelle.

L'intoxication chronique au D-Galactose a fortement altéré l'apprentissage spatial, comparativement au groupe témoin négatif (solution saline/ véhicule).

Le complément A2 n'a montré aucun effet sur le comportement d'alternance.

Le complément A3 a très significativement mais partiellement atténué les déficits induits par l'intoxication chronique D-Galactose.

Le complément A4 a très significativement et complètement atténué les déficits induits par l'intoxication chronique D-Galactose.

Le DHA seul selon A5 a très significativement mais partiellement allégé les déficits induits par l'intoxication chronique au D-Galactose.

De manière étonnante, il apparait que le traitement préventif par le complément de l'invention à la dose A4 a un effet positif supérieur (très significatif et atténuation complète des déficits) comparativement au traitement au DHA seul (très significatif et atténuation partielle des déficits), et ce pour une même dose de DHA.

Effets du complément et du DHA sur les déficits d'apprentissage induits par le D-Galactose

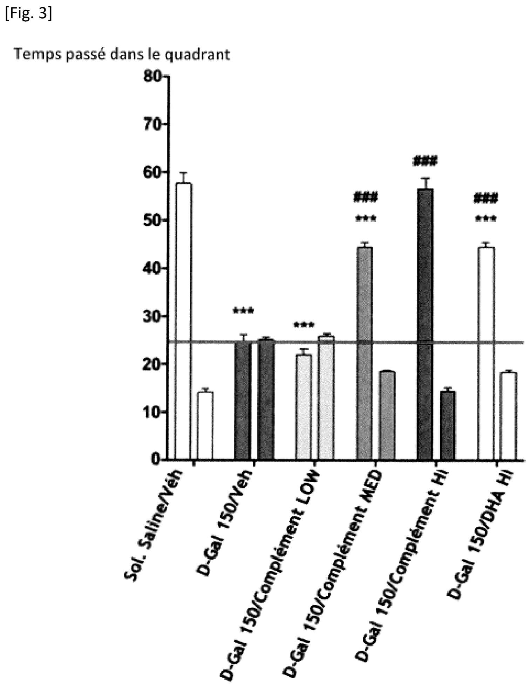

Les résultats sont représentés à la figure 3.

Sur la Figure 3 : LOW, faible dose du complément (A2) ; MED, dose moyenne du complément (A3) ; HI, dose élevée du complément (A4) ; N est compris entre 9 et 10 selon le groupe ; *** $p < 0,0001$ vs. solution saline / groupe Veh, /Veh; ### p < 0,0001 vs. groupe D-GAL 150 / Veh; test de comparaison multiple de Bonferroni après ANOVA bidirectionnelle.

L'intoxication chronique au D-Galactose a fortement altéré l'apprentissage spatial, comparativement au groupe témoin négatif (solution saline/ véhicule).

Le complément A2 n'a montré aucun effet sur le comportement d'alternance.

Le complément A3 a très significativement mais partiellement atténué les déficits induits par l'intoxication chronique D-Galactose.

Le complément A4 a très significativement et complètement allégé les déficits induits par l'intoxication chronique D-Galactose.

Le traitement par DHA seul selon A5 a très significativement mais partiellement allégé les déficits induits par l'intoxication chronique au D-Galactose.

De manière étonnante, il apparait que le traitement préventif par le complément de l'invention à la dose A4 a un effet positif supérieur (très significatif et atténuation complète des déficits) comparativement au traitement au DHA seul (très significatif et atténuation partielle des déficits), et ce pour une même dose de DHA. De plus, le traitement préventif avec le complément à la dose A3 a un effet identique (très significatif et atténuation partielle des déficits) au traitement par DHA seul alors que ce dernier est deux fois plus concentré en DHA.

Effets sur les déficits d'évitements passifs induits par le D-Galactose chez la souris

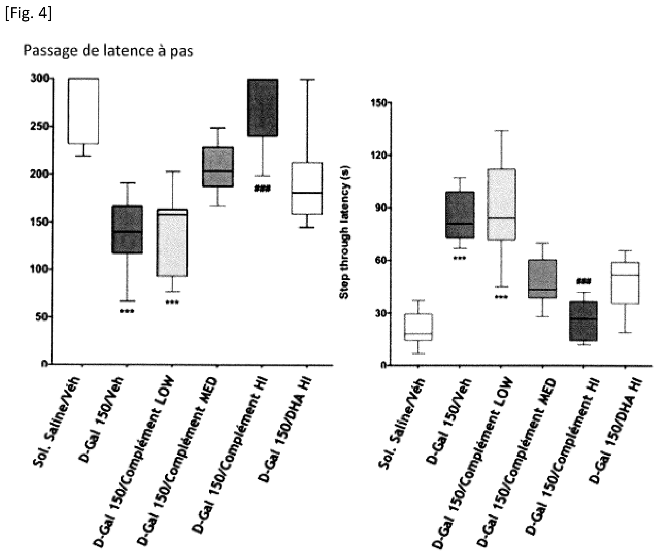

Les résultats sont représentés à la figure 4, avec les effets du complément de l'invention sur la latence de passage à pas illustrés sur le diagramme de gauche et sur la latence d'échappement illustrés sur le diagramme de droite, mesurés pendant la période de rétention.

Sur la figure 4 : LOW, faible dose du complément (A2) ; MED, dose moyenne du complément (A3) ; HI, dose élevée du complément (A4). N est compris entre 9 et 10 selon les groupes ; *** p < 0,0001 vs. solution saline / groupe Veh, ### p < 0,0001 vs. groupe D-GAL 150 / groupe Veh; test de Dunnett.

L'intoxication chronique au D-Galactose a fortement altéré la mémoire de travail contextuelle à long terme, comparativement au groupe témoin négatif (solution saline/ véhicule).

Le complément A2 n'a montré aucun effet sur la mémoire contextuelle à long terme.

Le complément A3 a permis d'atténuer de façon non significative les déficits induits par l'intoxication chronique au D-Galactose.

Le complément A4 très significativement et complètement atténué les déficits induits par l'intoxication chronique D-Galactose.

Le traitement au DHA seul (selon A5) a permis d'atténuer de façon non significative les déficits induits par l'intoxication chronique au D-Galactose.

De manière étonnante, il apparait que le traitement préventif par le complément de l'invention à la dose A4 a un effet positif supérieur (très significatif et atténuation complète des déficits) comparativement au traitement au DHA seul (atténuation non significative des déficits), et ce pour une même dose de DHA. De plus, le traitement préventif avec le complément à la dose A3 a un effet identique (atténuation non significative des déficits) au traitement par DHA seul alors que ce dernier est deux fois plus concentré en DHA.

Effets du complément et du DHA sur la peroxydation lipidique induite par le D-Galactose

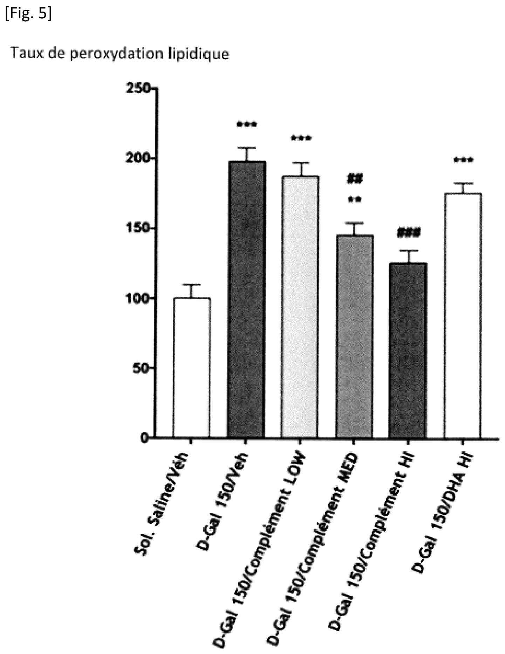

Les résultats sont montrés à la figure 5.

Sur la figure 5 : LOW, faible dose du complément (A2) ; MED, dose moyenne du complément (A3); HI, dose élevée du complément (A4). N est compris entre 9 et 10 selon les groupes ; ** p < 0,01, *** p < 0,0001 vs. la solution saline / groupe Veh, ## p < 0,01, ### p < 0,0001 vs. le groupe D-GAL 150 / groupe Veh; test de Dunnett.

L'intoxication chronique au D-Galactose a fortement augmenté le stress oxydatif comparativement au groupe témoin négatif (solution saline/ véhicule).

Le complément A2 n'a montré aucun effet sur la peroxydation lipidique induit par l'intoxication chronique D-Galactose.

Le complément A3 a très significativement mais partiellement réduit le stress oxydatif induit par l'intoxication chronique D-Galactose.

Le complément A4 très significativement et complètement réduit le stress oxydatif induit par l'intoxication chronique D-Galactose.

Le traitement au DHA seul selon A5 n'a montré aucun effet sur le stress oxydatif induit par l'intoxication chronique au D-Galactose.

De manière étonnante, il apparait que le traitement préventif avec le complément de l'invention à la dose A4 a un effet positif supérieur (très significatif et atténuation complète du stress oxydatif) comparativement au traitement au DHA seul, et ce pour une même dose de DHA.

Effets du complément et du DHA sur l'expression induite de TNF-$\alpha$ dans le cortex et le plasma par le D-Galactose

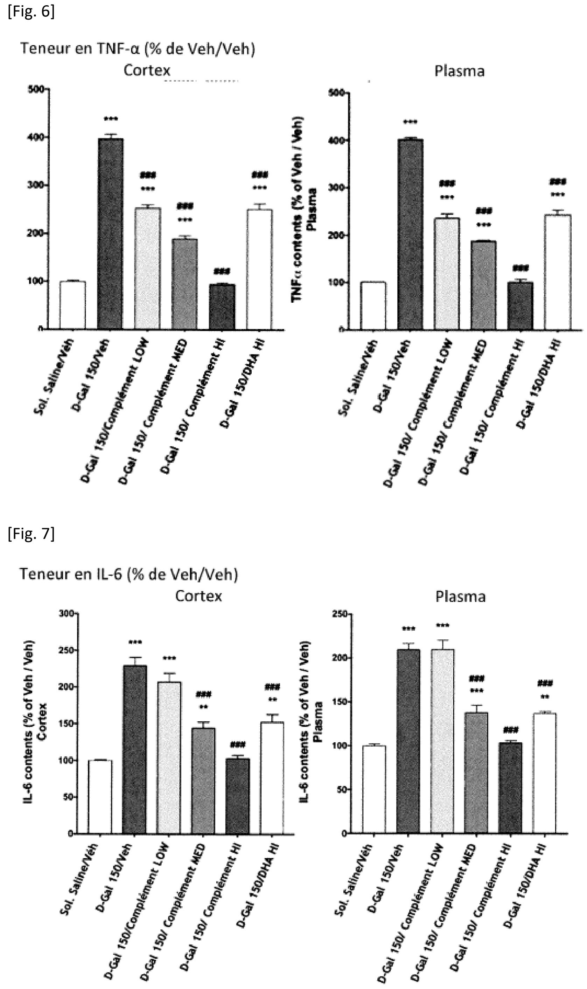

Les résultats sont représentés à la Figure 6, avec l'effet sur le cortex sur le diagramme de gauche et l'effet sur le plasma sur le diagramme de droite.

Sur la Figure 6 : LOW, faible dose du complément (A2) ; MED, dose moyenne du complément (A3) ; HI, dose élevée du complément (A4) ; N est compris entre 9 et 10 selon les groupes ; *** p < 0,0001 vs. la solution saline / groupe Veh, ### p < 0,0001 vs. le groupe D-GAL 150 / groupe Veh; test de Dunnett.

L'intoxication chronique au D-Galactose a fortement augmenté de façon significative le TNF-$\alpha$ dans le cortex et le plasma, comparativement au groupe témoin négatif (solution saline/véhicule).

Le complément A2 et A3 a très significativement mais partiellement réduite l'augmentation du TNF-$\alpha$ induite par l'intoxication chronique au D-Galactose dans le cortex et le plasma.

Le complément A4 a très significativement et complètement réduit le niveau de TNF-$\alpha$ dans le cortex et le plasma.

Le traitement au DHA seul selon A5 a très significativement mais partiellement réduite l'augmentation du TNF-$\alpha$ induite par l'intoxication chronique au D-Galactose dans le cortex et le plasma.

De manière étonnante, il apparait que le traitement préventif complément HI (A4) a un effet positif supérieur (très significatif et atténuation complète de l'augmentation de TNF-$\alpha$ dans le cortex et dans le plasma) comparativement au traitement au DHA seul, et ce pour une même dose de DHA. De plus, les traitements préventifs avec le complément aux doses A2 et A3 ont des effets identiques dans le cas du cortex et du plasma (atténuations très significatives des augmentations) au traitement par DHA seul alors que ce dernier est respectivement six et deux fois plus concentré en DHA comparativement aux traitements préventifs avec le complément aux doses A2 et A3, respectivement.

Effets du complément et du DHA sur l'expression induite d'IL-6 dans le cortex et le plasma par le D-Galactose

Les résultats sont représentés à la Figure 7, avec l'effet dans le cortex sur le diagramme de gauche et dans le plasma sur le diagramme de droite

Sur la Figure 7 :. LOW, faible dose du complément (A2) ; MED, dose moyenne du complément (A3) ; HI, dose élevée du complément (A4) ; N est compris entre 9 et 10 selon les groupes ; *** p < 0,0001 vs. la solution saline / groupe Veh, ### p < 0,0001 vs. le groupe D-GAL 150 / groupe Veh; test de Dunnett.

L'intoxication chronique au D-Galactose a fortement augmenté de façon significative l'IL-6 dans le cortex et le plasma, comparativement au groupe témoin négatif (solution saline/véhicule)

Le complément à faible dose (A2) n'a montré aucun effet sur la concentration d'IL-6 induite par l'intoxication au D-Galactose.

Le complément à dose moyenne (A3) a réduit très significativement mais partiellement l'augmentation du taux d'IL-6 induite par l'intoxication chronique D-Galactose dans le cortex et le plasma.

Le complément à forte dose (A4) a très significativement et complètement réduit le taux d'IL-6 dans le cortex et le plasma, induite par l'intoxication chronique D-Galactose dans le cortex et le plasma.

Le traitement au DHA seul selon A5 a réduit très significativement, mais partiellement, l'augmentation du taux d'IL-6 dans le cortex et dans le plasma, induite par l'intoxication chronique au D-Galactose.

De manière étonnante, il apparait que le traitement préventif complément HI selon A4 a un effet positif supérieur (très significatif et atténuation complète de l'augmentation de l'IL-6 dans le cortex et dans le plasma) comparativement au traitement au DHA seul (très significatif et atténuation partielle de l'augmentation de l'IL-6 dans le cortex et dans le plasma), et ce pour une même dose de DHA. De plus, le traitement préventif avec le complément à la dose A3 a un effet identique dans le cas du cortex (atténuation non significative des déficits) au traitement par DHA seul alors que ce dernier est deux fois plus concentré en DHA.

En conclusion :

L'intoxication chronique au D-Galactose induit de manière très significative une altération de la mémoire de travail spatiale, de la mémoire contextuelle à long terme et a compromis l'apprentissage spatial. Les altérations comportementales sont également liées à des altérations biochimiques en termes d'augmentation du stress oxydatif et d'induction de processus neuro-inflammatoires.

Le traitement préventif avec le complément de l'invention est dose-dépendant et, a, de manière très significative, complètement atténué dans le cas de la plus forte dose testée (complément A4) les déficits induits par l'intoxication chronique au D-Galactose en termes d'altération comportementale, d'augmentation du stress oxydatif, et d'activation des processus neuro-inflammatoires.

Le traitement au DHA seul (A5), et à dose équivalente en DHA comparativement au traitement complément HI, a réduit très significativement, mais partiellement les déficits induits par l'intoxication chronique au D-Galactose en termes d'altération comportementale, d'augmentation du stress oxydatif, et d'activation des processus neuro-inflammatoires.

De manière étonnante, le traitement préventif par le complément est significativement plus efficace à dose équivalente de DHA que le traitement préventif par DHA seul, vis-à-vis de l'atténuation du déclin cognitif lié à l'âge sur modèle murin causée par une intoxication chronique au D-Galactose. De plus, le traitement préventif par le complément, pour des doses deux fois inférieures en DHA, a des effets identiques positifs au traitement par DHA seul, alors que ce dernier est deux fois plus concentré en DHA, dans le cas de la diminution du stress oxydatif mesuré dans le cortex (IL-6 et TNF-$\alpha$), dans le plasma (TNF-$\alpha$), dans le cas de la mémoire contextuelle à long terme et dans le cas de l'apprentissage spatial. Et, le traitement préventif par le complément a des effets identiques positifs au traitement par DHA seul, alors que ce dernier est six fois plus concentré en DHA, dans le cas de la diminution du stress oxydatif mesuré dans le cortex (IL-6 et TNF-$\alpha$) et le plasma (TNF-$\alpha$).

Ainsi, en appliquant la formule de calcul de dose équivalente journalière chez l'humain, un traitement préventif du déclin cognitif lié à l'âge peut être défini avec la prise journalière de 2 à 5 mg de complément/kg de poids corporel.

**Exemple 3** : **Test d'un extrait de la microalgue *Tisochrysis lutea* chez de jeunes rats femelles atteintes par un stress prénatal**

Dans cet exemple, sont étudiés la résolution des déficits cognitifs, le comportement anxieux et l'altération de la mémoire de reconnaissance, induits chez de jeunes rats femelles après un stress prénatal de l'ascendance, *via* l'administration d'un complément à base d'un extrait de la microalgue *Tisochrysis lutea* correspondant à celui utilisé dans l'Exemple 2.

Matériel et méthodes

Le modèle utilisé dans cet exemple est un modèle reconnu d'induction du stress prénatal chez le rat par immobilisation de la femelle en gestation dans un cylindre sous un éclairage violent.

Des rats femelles gravides ont été assignées au hasard à des groupes de stress prénatal (SP) ou à des groupes témoins (NS), logées individuellement dans des cages d'élevage en plastique, et ont eu accès *ad libitum* à de la nourriture et à de l'eau à l'exception des moments des tests comportementaux. Les conditions au sein des cages sont les suivantes : photopériode de 12 h de lumière /12 h de cycle sombre (lumière allumée à 07h00), dans une pièce constante à température (21°C) et humidité (50%) constante.

La procédure de stress prénatal a été effectuée comme décrit par Meunier et col. (2004). Les immobilisations des rats femelles ont fait l'objet d'une procédure de contrainte semi-aléatoire. Les animaux ont été placés et retenus dans des cylindres transparents en plexiglas (20 cm de long, 7 cm de diamètre) sous une lumière vive pendant une durée totale de 90 minutes par jour, pendant 4 jours consécutifs. Pour que le stress soit le plus imprévisible possible, la période d'immobilisation forcée de 90 minutes a été administrée de la façon suivante : une unique phase de 90 min, deux phases de 45 min espacées de 4 h, deux phases de 60 et 30 min espacées de 4 h, ou trois phases de 30 min espacées de 4 et 1 h, et ce, à différents moments de la journée.

Des mères témoins ont également été manipulées, mais n'ont jamais été placées dans les cylindres de contention.

Il a été permis aux rats femelles traitées de se libérer naturellement de l'entrave des cylindres de contention à partir du jour 1 après la naissance (JPN1).

Les portées ont été sevrées au JPN21. Les rats ont été séparés des mères, identifiés en fonction de leur sexe, pesées et réparti par même sexe au sein de cages (3 rats par cage). Les jeunes rats au sein d'une même cage provenaient de portées différentes, afin d'éviter tout effet possible lié à la portée.

Les conditions au sein des cages sont les suivantes : photopériode de 12h de lumière /12h de cycle sombre (lumière allumée à 07h00), dans une pièce constante à température (21°C) et humidité (50%) constante, avec accès *ad libitum à* la nourriture et à l'eau, sauf pendant les expériences comportementales.

Dans chaque cage, les animaux ont reçu le même traitement. Les animaux ont été testés de manière aléatoire et en double aveugle.

Quarante-huit (48) rats femelles ont été utilisés et regroupés en quatre groupes d'animaux, constitués de la manière suivante :

Le groupe 1 est composé de 12 rats femelles naïves, c'est-à-dire dont l'ascendance n'a pas subi de stress prénatal, et recevant uniquement 200 $\mu$L par jour de solution véhicule (référence : NS/Véhicule). Ce groupe est donc le groupe témoin ;

Le groupe 2 est composé de 12 rats femelles naïves, c'est-à-dire dont l'ascendance n'a pas subi de stress prénatal, et recevant 200 $\mu$L par jour de complément (référence : NS/Complément) ;

Le groupe 3 est composé de 12 rats femelles dont l'ascendance a subi de stress prénatal, et recevant 200 $\mu$L par jour

de solution véhicule (référence : SP/Véhicule) ;

Le groupe 4 est composé de 12 rats femelles dont l'ascendance a subi de stress prénatal, et recevant 200 µL par jour du complément (référence : SP/Complément).

L'efficacité du complément a été évaluée 6 semaines après la naissance.

Le complément (une dose) a été administré par gavage une fois par jour, 5 jours par semaine. Les administrations ont commencé après le sevrage, soit après le jour postnatal (JPN) 25, et a duré jusqu'au JPN46.

L'apport journalier était de 25,7 mg de complément par kg de poids corporel de rat.

Les animaux ont été soumis à des tests de comportement durant la période entre les jours JPN46 et JPN48, soit hors de la période de traitement par le véhicule ou le complément. Les effets qui sont donc observés lors des tests de comportements seront donc dus à un traitement de nature préventive.

Les tests comportementaux sont décomposés en une session d'évaluation de l'anxiété et de deux sessions de reconnaissances d'objets. Les séances sont définies comme suit :

- Session 1, JPN 46 : Les rats ont été placés individuellement dans un espace ouvert carré (50 cm x 50 cm x 50 cm x 50 cm) en plexiglas bleu avec un plancher équipé de diodes électroluminescentes infrarouges. Les rats ont été habitués à l'espace de test pendant une séance de 10 minutes et leurs déplacements capturés par une caméra infrarouge et analysés grâce au logiciel Ethovision® (Noldus). L'activité a été analysée en fonction de la distance totale parcourue (m), et en fonction du pourcentage de présence dans la zone centrale de 25 x 25 cm définie par le logiciel. Ces données rendent compte de l'intensité du comportement anxieux [38].

- Session 2 JPN 47 : Deux objets identiques (tube Eppendorf en plastique de 50 mL) ont été placés à des endroits définis (sur deux bords opposés de la zone centrale). Chaque rat a été placé au sein de l'espace de test et l'activité exploratoire enregistrée pendant une session de 10 minutes. L'activité était analysée en termes de nombre de contacts avec les objets et de la durée des contacts.

- Session 3 JPN 48 : L'objet de la session 2 a été remplacé par un nouvel objet (un bouchon de bouteille en plastique) dont la forme, la texture, la couleur diffèrent de celles de l'objet familier. Chaque rat a été replacé dans l'espace de test et l'activité exploratoire enregistrée au cours d'une séance de 10 minutes. L'activité a fait l'objet d'une analyse similaire à celle décrite dans la séance 2.

L'indice d'exploration préférentielle a été calculé comme étant le ratio du nombre (ou durée) de contacts avec l'objet de la session 2, sur le nombre total (ou durée) des contacts avec les deux objets.

Toutes les valeurs sont exprimées en moyenne plus ou moins l'écart type de mesure. Des analyses statistiques sont effectuées séparément pour chaque composé à l'aide d'une ANOVA unidirectionnelle (valeur F), suivies du test de comparaison multiple post-hoc de Dunnett.

Le calcul de la dose journalière équivalente chez l'humain, à partir de la dose journalière testée chez le rat est défini comme suit par la FDA (Guidance, 2005) : la dose journalière chez l'humain exprimée en mg/kg de poids corporel (HED Human) est égale à la dose journalière chez l'animal exprimée en mg/kg de poids corporel (HED Animal) multiplié par le ratio du facteur de sécurité (Km Animal) chez l'animal considéré et du facteur de sécurité pour l'homme (Km Human). Le Km Humain est égal à 37 et le Km Rat est égal à 6.

Les résultats sont présentés ci-après.

Locomotion au centre de l'espace de test, jour JPN46 ; effet du complément sur l'anxiété

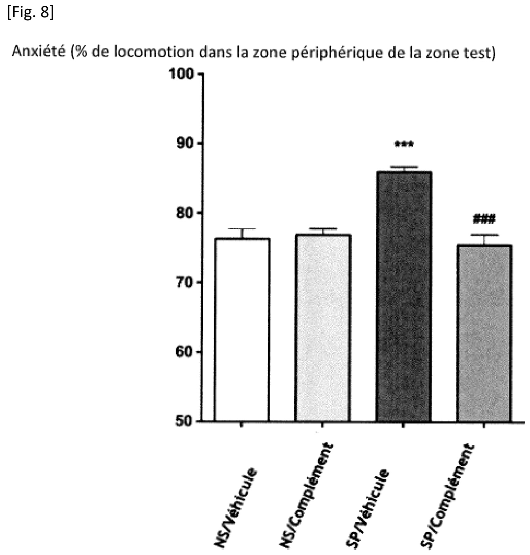

Les résultats sont représentés à la Figure 8.

A la Figure 8 : Effets des traitements sur l'anxiété. N = 12 ; *** p < 0,0001 par rapport au groupe NS / véhicule traité ; #### p < 0,0001 par rapport au groupe SP / véhicule traité ; test de Dunnett

Le groupe SP/véhicule, correspondant aux individus ayant subis un stress prénatal et traités préventivement uniquement avec le véhicule, ont un pourcentage de déplacements très significativement plus élevé au sein de la zone périphérique de l'espace de test ouvert comparativement au groupe NS/Véhicule (groupe n'ayant pas subi de stress prénatal).

Le groupe SP/complément, correspondant aux individus ayant subis un stress prénatal et traités préventivement avec le complément ont un pourcentage de déplacements très significativement plus bas au sein de la zone périphérique de l'espace de test ouvert comparativement au groupe SP/Véhicule (groupe ayant subi un stress prénatal et non traité avec le complément). De plus, le pourcentage de déplacements du groupe SP/complément est équivalent à celui du groupe témoin NS/véhicule.

Un taux de déplacements des individus dans la zone périphérique de l'espace de test ouvert supérieur à la modalité témoin démontre un comportement anxieux [63], *via* un mécanisme de protection basé sur la recherche des bordures limitant les zones à découvert et à surveiller.

Ainsi, le stress prénatal (SP) a induit un comportement d'anxiété très significatif.

De manière étonnante, il apparait que le complément a très significativement et entièrement atténué le comportement anxieux induit par le stress prénatal.

Test de reconnaissance, jour JPN47 ; effet du complément sur la mémoire de reconnaissance sur la reconnaissance d'un objet

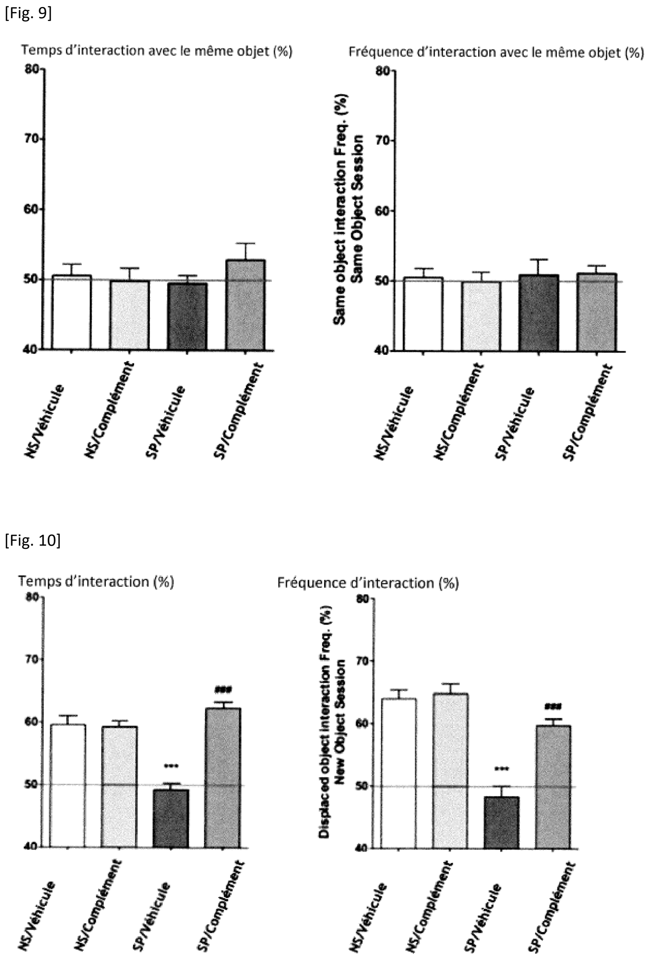

Les résultats sont représentés à la Figure 9.

Lors de cette session le même objet est présenté deux fois aux individus.

Aucun effet statistique entre les groupes n'a été mesuré pour ce paramètre.

Ainsi, les individus de l'ensemble des groupes ont interagi de manière équivalente lors de la mise en contact des objets identiques et leurs interactions, tant en termes de fréquence que de durée sont également réparties entre les deux objets (50 %).

Test de reconnaissance, jour JPN48 (nouvel objet) ; effet du complément sur la mémoire de reconnaissance pour le test de reconnaissance d'un nouvel objet

Les résultats sont représentés à la Figure 10.

A la Figure 10 : N = 12 ; *** p < 0,0001 par rapport au groupe NS / véhicule traité ; ### p < 0,0001 par rapport au groupe SP / véhicule traité ; test de Dunett.

Lors de cette session deux objets différents sont présentés une fois chacun aux individus : un des objets correspond à l'objet présenté lors de la session 2 et l'autre objet est un nouvel objet.

Le groupe SP/véhicule, correspondant aux individus ayant subis un stress prénatal et traités préventivement uniquement avec le véhicule, ont un pourcentage d'interactions, tant en fréquence qu'en durée, avec le nouvel objet présenté très significativement plus bas comparativement au groupe NS/Véhicule (groupe n'ayant pas subi de stress prénatal). Et ce pourcentage est égal à celui de la session 2 obtenu pour l'ensemble des groupes. Ainsi les individus du groupe SP/véhicule ont autant d'interactions avec l'ancien qu'avec le nouvel objet et donc les individus de ce groupe ne reconnaissent pas l'ancien objet présenté lors de la session 2.

*A contrario,* le groupe SP/Complément correspondant aux individus ayant subis un stress prénatal et traités préventivement avec le complément, ont un pourcentage d'interactions, tant en fréquence qu'en durée, avec le nouvel objet présenté très significativement plus élevé comparativement au groupe PS/Véhicule (groupe témoin négatif). Et ce pourcentage est supérieur à celui de la session 2 obtenu pour l'ensemble des groupes. Ainsi, les individus du groupe SP/Complément ont moins d'interactions avec l'ancien objet qu'avec le nouvel objet, et donc les individus de ce groupe reconnaissent l'ancien objet présenté lors de la session 2. De plus, les individus du groupe SP/Complément ont un pourcentage d'interactions, tant en fréquence qu'en durée, avec le nouvel objet présenté équivalent avec ceux des groupes NS/Véhicule et NS/Complément.

Ainsi, le stress prénatal (SP) a induit des déficits de mémoire de reconnaissance très importants dans le cas du nouvel objet.

De manière étonnante, il apparait que le complément a permis d'atténuer très significativement et complètement les déficits de mémoire de reconnaissance induits par le stress prénatal.

En conclusion :

Le traitement au complément a, de manière très significative, complètement atténué le comportement anxieux ainsi que les déficits de mémoire de reconnaissance induits par le stress prénatal.

Le stress prénatal tel que pratiqué dans cette expérimentation induit de manière très significative un comportement anxieux, et altère de manière très significative la mémoire de reconnaissance chez les jeunes rats femelles.

Ainsi, en appliquant la formule de calcul de dose équivalente journalière chez l'humain, un traitement préventif atténuant les troubles cognitifs causés par un stress prénatal peut être défini avec la prise journalière de 0,05 à 0,1 mg de complément/kg de poids corporel.

**Exemple 4: Test d'un extrait naturel de la microalgue *Phaeodactylum tricornutum* dans le cadre du modèle *in vivo* sur l'atténuation des déficits induits par le déclin cognitif lié à l'âge**

Le complément alimentaire de l'invention est préparé à partir d'un extrait de *Phaeodactylum tricornutum* qui comprend en mg/g :

Acides gras de type oméga-3 (ALA, SDA, EPA, DHA) : 66,6 ± 11,5 ;
Fucoxanthine : 20,0 ± 4,0 ;
Stérols : 3,0 ± 0,6 ;

Phycoprostane : 0,0025 ± 0,0005

Le complément est obtenu par addition d'huile de noix de coco à raison de 410 mg ± 20 mg/g audit extrait.

Le complément est incorporé à des croquettes selon 4 formulations différentes telles que les quantités de complément incorporées au sein des différents lots de croquettes correspondent à des doses journalières en équivalent humain telles que décrites dans le tableau 2, et ce, par dilution de la composition décrite ci-dessous dans de l'huile de noix de coco à masse finale égale entre les formulations.

Le calcul de la dose journalière équivalente chez l'humain, à partir de la dose journalière testée chez la souris est défini comme suit par la FDA (Guidance, 2005) : la dose journalière chez l'humain exprimée en mg/kg de poids corporel (HED Human) est égale à la dose journalière chez l'animal exprimée en mg/kg de poids corporel (HED Animal) multipliée par le ratio du facteur de sécurité (Km Animal) chez l'animal considéré et du facteur de sécurité pour l'homme (Km Humain). Le Km Humain est égal à 37 et le Km Souris est égal à 3.

Un lot supplémentaire de croquettes est formulé uniquement avec l'huile de noix de coco, tel que la concentration en véhicule soit équivalente à celle des autres lots, soit 0,01 % (m:m).

Les cinq lots de croquettes ainsi obtenus sont référencés tels que décrit dans le tableau 2 ci-dessous.

[Table 2]

| Croquettes formulées | Dose en complément en équivalent humain (mg de complément/kg de masse corporelle/jour) | Référence |
|---|---|---|
| Huile de noix de coco (véhicule) | 0 | Veh. |
| Complément | 1,7 | D1 |
| Complément | 3,3 | D2 |
| Complément | 4,2 | D3 |
| Complément | 5,3 | D4 |

Le modèle *in vivo* considéré est le modèle D-Galactose appliqué à des souris qui est adapté à l'étude du déclin cognitif lié à l'âge. Ce modèle imite en effet de nombreuses caractéristiques comportementales et moléculaires du vieillissement cérébral dans les modèles de rongeurs.

Le D-Galactose est administré par voie sous-cutanée à raison de 150 mg/kg de poids frais de souris par jour, et le complément alimentaire ci-dessus est incorporé dans un granulé, selon le schéma suivant :

- Entre le jour -28 et le jour 51, le complément est administré par incorporation dans des granulés alimentaires ;
- Entre le jour 01 et le jour 51, le D-Galactose est administré par voie sous-cutanée, cinq jours par semaine ;
- Entre les jours 43 et 51, trois tests comportementaux différents sont utilisés pour surveiller les effets des composés d'essai.

L'efficacité du complément est évaluée selon les paramètres suivants : amélioration des déficits d'apprentissage (mémoire de travail spatiale : alternance spontanée dans le labyrinthe en Y selon le test Y-maze ; mémoire spatiale par le test dit « Morris Water Maze » et mémoire contextuelle à long terme dans le test d'évitement passif), taux de peroxydation lipidique (LPO) dans l'hippocampe et l'effet sur les marqueurs de neuro-inflammation IL6 et TNFα.

Amélioration des déficits d'apprentissage

- Au jour 43, tous les animaux ont été testés pour la performance d'alternance spontanée dans le test Y-maze (YM), via un indice de mémoire de travail spatiale ;
- Du jour 44 jusqu'au jour 49, tous les animaux ont été testés pour la mémoire spatiale dans le test Morris Water Maze (MWM), via un indice de mémoire spatiale ;
- Du jour 44 jusqu'au jour 49, tous les animaux sont testés via le test MWM pour évaluer la mémoire de travail spatiale ;
- Les jours 50 et 51, la mémoire contextuelle à long terme des animaux est évaluée à l'aide de la procédure d'évitement passif de type pas-à-pas (STPA), via des séances d'entraînement et de rétention, respectivement ;

Taux de peroxydation lipidique (LPO) dans l'hippocampe et effet sur les marqueurs de neuro-inflammation IL6 et TNF$\alpha$

Le 51e jour, après les tests comportementaux, les animaux sont euthanasiés.

Pour tous les animaux, le sang du tronc est prélevé et centrifugé pour récupérer le plasma, et le cerveau est rapidement prélevé. L'hippocampe et le cortex sont disséqués, l'hippocampe est ensuite utilisé pour déterminer les taux de peroxydation lipidique par méthode colorimétrique ; le cortex hémi-frontal et le plasma sont utilisés pour déterminer le niveau des biomarqueurs inflammatoires interleukine-6 (IL-6) et le facteur alpha de nécrose tumorale (TNF-$\alpha$).

La quantification des taux de peroxydation lipidique (LPO) a été réalisée selon la procédure modifiée et adaptée de Hermes-Lima et col.. Cette méthode mesure la capacité des lipides peroxydés du cerveau à oxyder un complexe d'oxyde ferreux et de xylénol orange, mise en évidence en présence d'hydroperoxyde de cumène (CHP). Le niveau de peroxydation lipidique est déterminé en équivalent-CHP selon :

CHPE = A5801/A5802 $\times$ [CHP (nmol)] et exprimé en équivalent-CHP par poids humide de tissu et en pourcentage comparativement aux données obtenues pour le groupe témoin (D-Galactose + véhicule).

Les teneurs en IL6 et TNF$\alpha$ sont quantifiées au moyen de tests ELISA avec les kits suivants :

Pour la quantification de l'IL6 : ThermoScientifique, EM2IL6
Pour la quantification du TNF$\alpha$ : ThermoScientifique, EMTNFA

Pour tous les essais, le cortex est homogénéisé après décongélation dans un tampon de 50 mM Tris-150 mM NaCl, pH 7,5, et soniqué pendant 20 s. Après centrifugation (16 100 g pendant 15 min, 4 °C), un surnageant ou du plasma sont utilisés pour les essais ELISA conformément aux instructions du fabricant des tests ELISA. Pour chaque essai, l'absorbance est lue à 450 nm et la concentration de l'échantillon calculée à l'aide de la courbe étalon. Les résultats sont exprimés en pg de marqueur par mg de tissu frais.

Toutes les valeurs, à l'exception des latences d'évitement passif, sont exprimées en moyenne plus ou moins l'écart type de mesure. Des analyses statistiques sont effectuées séparément pour chaque composé à l'aide d'une ANOVA unidirectionnelle (valeur F), suivies du test de comparaison multiple post-hoc de Dunnett. Les latences d'évitement passives ne suivent pas une distribution gaussienne, puisque les temps limites supérieurs sont fixés. Elles sont donc analysées à l'aide d'une ANOVA non paramétrique Kruskal-Wallis (valeur H), suivie d'un test de comparaison multiple de Dunn. Les valeurs avec $p < 0,05$ sont considérées comme statistiquement significatives.

Les tests sont effectués sur 72 souris mâles, réparties dans 6 groupes de 12 souris, parmi lesquels le groupe 1 est le groupe témoin négatif et le groupe 2-6 est le groupe témoin positifs:

le groupe 1 est le groupe auquel est administré une solution saline sous cutanée au lieu du D-Galactose et des croquettes B1 ;
le groupe 2 est le groupe auquel sont administrés du D-Galactose et des croquettes B1 ;
le groupe 3 est le groupe auquel sont administrés du D-Galactose et des croquettes B2 ;
le groupe 4 est le groupe auquel sont administrés du D-Galactose et des croquettes B3 ; et
le groupe 5 est le groupe auquel sont administrés du D-Galactose et des croquettes B4 ; et
le groupe 6 est le groupe auquel sont administré du D-Galactose et des croquettes B5.

Effets sur la mémoire spatiale dans l'alternance spontanée du test Y-maze :

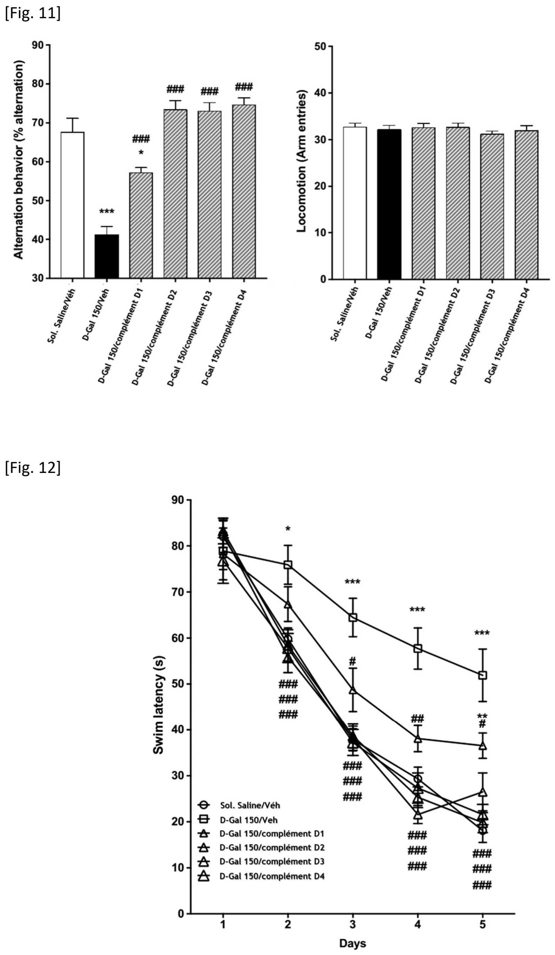

Les résultats sont représentés à la figure 11, le premier diagramme (à gauche) illustrant les effets du complément de l'invention sur les déficits d'alternances spontanées et le second diagramme (à droite) illustrant les effets du complément de l'invention sur l'activité locomotrice.

Sur la figure 11 : Sol. Saline/veh correspond au témoin négatif (groupe non traité au D-galactose et nourri avec les croquettes formulées avec le véhicule, huile de noix de Coco) ; DGal 150/Veh correspond au témoin positif (groupe traité au D-Galactose et nourri avec les croquettes formulées avec le véhicule, huile de noix de Coco) ; D1, D2, D3 et D4 doses croissantes du complément ; N est compris entre 11 et 12 selon les groupes ; * $p < 0,05$, *** $p < 0,0001$ vs. la solution saline / groupe Veh, # $p < 0,05$, ## $p < 0,01$, ### $p < 0,0001$ vs. le groupe D-GAL 150 / groupe Veh; test de Dunnett.

On observe que le traitement au D-Galactose a significativement altéré la mémoire de travail spatiale, comparativement aux souris traitées par solution saline.

Le complément D1 a très significativement mais partiellement atténué les déficits induits par l'intoxication chronique au D-Galactose. Les compléments D2, D3 et D44 ont très significativement et complètement atténués les déficits induits par l'intoxication chronique au D-Galactose.

Effets sur les déficits d'apprentissage induits par le D-Gal selon le test MWM :

Les résultats sont représentés à la figure 12.

Sur la Figure 12 : Sol. Saline/veh correspond au témoin négatif (groupe non traité au D-galactose et nourri avec les croquettes formulées avec le véhicule, huile de noix de Coco) ; DGal 150/Veh correspond au témoin positif (groupe traité au D-Galactose et nourri avec les croquettes formulées avec le véhicule, huile de noix de Coco) ; D1, D2, D3 et D4 doses croissantes du complément; N est compris entre 11 et 12 selon les groupes ; * $p < 0,05$, ** $p < 0,01$, *** $p < 0,0001$ vs. solution saline / groupe Veh, ## $p < 0,01$, ### $p < 0,0001$ vs. groupe D-GAL 150 / groupe Veh; le test de comparaison multiple de Bonferroni après ANOVA bidirectionnelle.

L'intoxication chronique au D-Galactose a fortement altéré l'apprentissage spatial, comparativement au groupe témoin négatif (solution saline/ véhicule).

Le complément D1 a très significativement mais partiellement atténué les déficits induits par l'intoxication chronique D-Galactose.

Les compléments D2, D3 et D4 ont très significativement et complètement atténués les déficits induits par l'intoxication chronique D-Galactose.

Effets du complément sur les déficits d'apprentissage induits par le D-Galactose

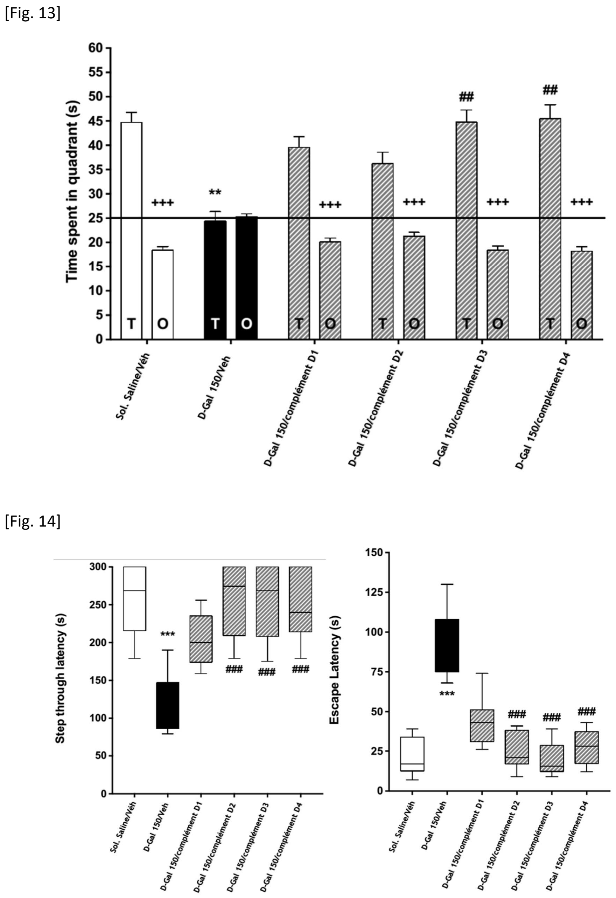

Les résultats sont représentés à la figure 13.

Sur la Figure 13 : Sol. Saline/veh correspond au témoin négatif (groupe non traité au D-galactose et nourri avec les croquettes formulées avec le véhicule, huile de noix de Coco) ; DGal 150/Veh correspond au témoin positif (groupe traité au D-Galactose et nourri avec les croquettes formulées avec le véhicule, huile de noix de Coco) ; D1, D2, D3 et D4 doses croissantes du complément; N est compris entre 11 et 12 selon les groupes ; Sol. Saline/veh correspond au témoin négatif (groupe non traité au D-galactose et nourri avec les croquettes formulées avec le véhicule, huile de noix de Coco) ; DGal 150/Veh correspond au témoin positif (groupe traité au D-Galactose et nourri avec les croquettes formulées avec le véhicule, huile de noix de Coco) ; D1, D2, D3 et D4 doses croissantes du complément ; N est compris entre 11 et 12 selon les groupes ; *** $p < 0,0001$ vs. solution saline / groupe Veh, /Veh; ### $p < 0,0001$ vs. groupe D-GAL 150 / Veh; test de comparaison multiple de Bonferroni après ANOVA bidirectionnelle. "T", temps passé dans le quadrant cible ; "O", temps moyen passé dans les trois autres quadrants.

L'intoxication chronique au D-Galactose a fortement altéré l'apprentissage spatial, comparativement au groupe témoin négatif (solution saline/ véhicule).

Les compléments D1 et D2 ont très significativement mais partiellement atténués les déficits induits par l'intoxication chronique D-Galactose.

Les compléments D3 et D4 ont très significativement et complètement allégés les déficits induits par l'intoxication chronique D-Galactose.

Effets sur les déficits d'évitements passifs induits par le D-Galactose chez la souris

Les résultats sont représentés à la figure 14, avec les effets du complément de l'invention sur la latence de passage à pas illustrés sur le diagramme de gauche et sur la latence d'échappement illustrés sur le diagramme de droite, mesurés pendant la période de rétention.

Sur la figure 14 : Sol. Saline/veh correspond au témoin négatif (groupe non traité au D-galactose et nourri avec les croquettes formulées avec le véhicule, huile de noix de Coco) ; DGal 150/Veh correspond au témoin positif (groupe traité au D-Galactose et nourri avec les croquettes formulées avec le véhicule, huile de noix de Coco) ; D1, D2, D3 et D4 doses croissantes du complément ; N est compris entre 11 et 12 selon les groupes ; *** $p < 0,0001$ vs. solution saline / groupe Veh, ### $p < 0,0001$ vs. groupe D-GAL 150 / groupe Veh; test de Dunnett.

L'intoxication chronique au D-Galactose a fortement altéré la mémoire de travail contextuelle à long terme, comparativement au groupe témoin négatif (solution saline/ véhicule).

Le complément D1 n'a montré aucun effet sur la mémoire contextuelle à long terme.

Les compléments D2, D3 et D4 ont très significativement et complètement atténués les déficits induits par l'intoxication chronique D-Galactose.

Effets du complément sur la peroxydation lipidique induite par le D-Galactose

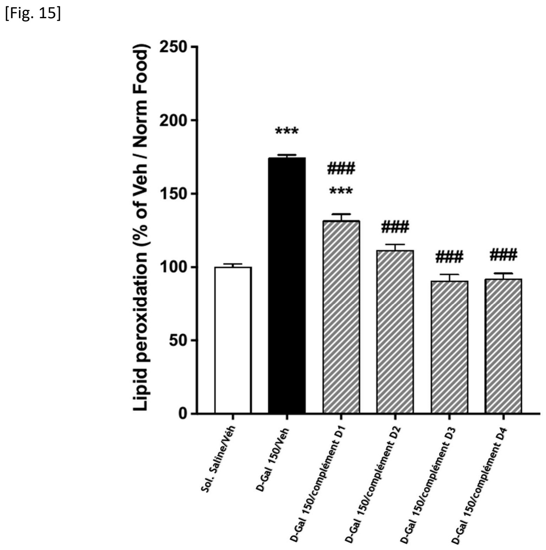

Les résultats sont montrés à la figure 15.

Sur la figure 15 : Sol. Saline/veh correspond au témoin négatif (groupe non traité au D-galactose et nourri avec les croquettes formulées avec le véhicule, huile de noix de Coco) ; DGal 150/Veh correspond au témoin positif (groupe traité au D-Galactose et nourri avec les croquettes formulées avec le véhicule, huile de noix de Coco) ; D1, D2, D3 et D4 doses croissantes du complément ; N est compris entre 11 et 12 selon les groupes ; ** p < 0,01, *** p < 0,0001 vs. la solution saline / groupe Veh, ## p < 0,01, ### p < 0,0001 vs. le groupe D-GAL 150 / groupe Veh; test de Dunnett.

L'intoxication chronique au D-Galactose a fortement augmenté le stress oxydatif comparativement au groupe témoin négatif (solution saline/ véhicule).

Le complément D1 a très significativement mais partiellement réduit le stress oxydatif induit par l'intoxication chronique D-Galactose.

Les compléments D2, D3 et D4 ont très significativement et complètement réduit le stress oxydatif induit par l'intoxication chronique D-Galactose.

Effets du complément sur l'expression induite de TNF-$\alpha$ dans le cortex et le plasma par le D-Galactose

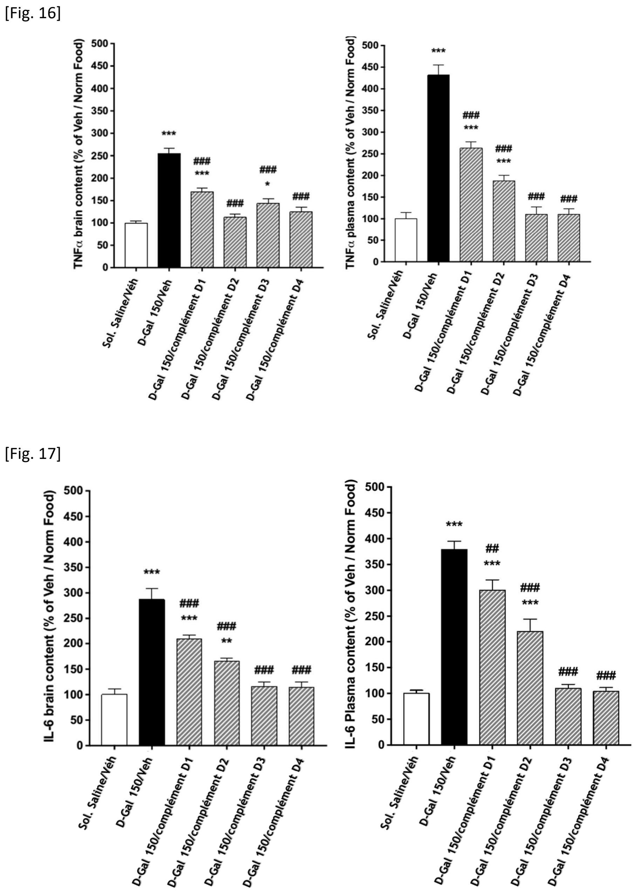

Les résultats sont représentés à la Figure 16, avec l'effet sur le cortex sur le diagramme de gauche et l'effet sur le plasma sur le diagramme de droite.

Sur la Figure 16 : Sol. Saline/veh correspond au témoin négatif (groupe non traité au D-galactose et nourri avec les croquettes formulées avec le véhicule, huile de noix de Coco) ; DGal 150/Veh correspond au témoin positif (groupe traité au D-Galactose et nourri avec les croquettes formulées avec le véhicule, huile de noix de Coco) ; D1, D2, D3 et D4 doses croissantes du complément; N est compris entre 11 et 12 selon les groupes ; *** p < 0,0001 vs. la solution saline / groupe Veh, ### p < 0,0001 vs. le groupe D-GAL 150 / groupe Veh; test de Dunnett.

L'intoxication chronique au D-Galactose a fortement augmenté de façon significative le TNF-$\alpha$ dans le cortex et le plasma, comparativement au groupe témoin négatif (solution saline/véhicule).

Le complément D1 a diminué de façon très significative mais partielle l'augmentation TNF-$\alpha$ induite par l'intoxication chronique au D-Gal dans le cerveau et le plasma.

Le complément D2 a réduit de façon très significative et complète l'augmentation de TNF-$\alpha$ induite par l'intoxication chronique au D-Gal dans le cerveau, mais partiellement dans les plasmas

Le complément D3 a diminué de façon très significative mais partielle l'augmentation TNF-$\alpha$ induite par l'intoxication chronique au D-Gal dans le cerveau, et entièrement dans le plasma.

Le complément D4 a réduit de façon très significative et complète l'augmentation de TNF-$\alpha$ induite par l'intoxication chronique au D-Gal dans le cerveau et le plasma.

Effets du complément sur l'expression induite d'IL-6 dans le cortex et le plasma par le D-Galactose

Les résultats sont représentés à la Figure 17, avec l'effet dans le cortex sur le diagramme de gauche et dans le plasma sur le diagramme de droite.

Sur la Figure 17 : Sol. Saline/veh correspond au témoin négatif (groupe non traité au D-galactose et nourri avec les croquettes formulées avec le véhicule, huile de noix de Coco) ; DGal 150/Veh correspond au témoin positif (groupe traité au D-Galactose et nourri avec les croquettes formulées avec le véhicule, huile de noix de Coco) ; D1, D2, D3 et D4 doses croissantes du complément; N est compris entre 11 et 12 selon les groupes ; *** p < 0,0001 vs. la solution saline / groupe Veh, ### p < 0,0001 vs. le groupe D-GAL 150 / groupe Veh; test de Dunnett.

L'intoxication chronique au D-Galactose a fortement augmenté de façon significative l'IL-6 dans le cortex et le plasma, comparativement au groupe témoin négatif (solution saline/véhicule)

Les compléments D1 et D2 ont réduits de façon très significative mais partielle l'augmentation de l'IL-6 induite par l'intoxication chronique au D-Gal dans le cerveau et les plasmas.

Les compléments D3 et D4 ont réduits de manière très significative et complète l'augmentation de l'IL-6 induite par l'intoxication chronique au D-Gal dans le cerveau et le plasma.

En conclusion :

L'intoxication chronique au D-Galactose induit de manière très significative une altération de la mémoire de travail spatiale, de la mémoire contextuelle à long terme et a compromis l'apprentissage spatial. Les altérations comportementales sont également liées à des altérations biochimiques en termes d'augmentation du stress oxydatif et d'induction de processus neuro-inflammatoires.

Le traitement préventif avec le complément de l'invention est dose-dépendant et, a, de manière très significative, complètement atténué dans le cas de la plus forte dose testée (complément D4) les déficits induits par l'intoxication chronique au D-Galactose en termes d'altération comportementale, d'augmentation du stress oxydatif, et d'activation des processus neuro-inflammatoires. Et dans les cas des doses inférieures intermédiaires (compléments D2 et D3), le complément de l'invention a, de manière très significative, complètement atténué les déficits induits par l'intoxication chronique au D-Galactose en termes de mémoire spatiale de travail, d'augmentation du stress oxydatif, et d'activation des processus neuro-inflammatoires.

Ainsi, en appliquant la formule de calcul de dose équivalente journalière chez l'humain, un traitement préventif du déclin cognitif lié à l'âge peut être défini avec la prise journalière de 1.7 à 5.3 mg de complément/kg de poids corporel.

Modes réalisations :

A. Composition comprenant au moins 50 mg/g d'un ou plusieurs acides gras de type oméga-3, au moins 10 mg/g d'un ou plusieurs xanthophylles, au moins 1 mg/g d'un ou plusieurs stérols et au moins 2 μg/g d'un ou plusieurs phycoprostanes.

B. Composition selon le mode A, caractérisée en ce qu'elle comprend 50 à 250 mg/g d'un ou plusieurs acides gras de type oméga-3, 10 à 50 mg/g d'un ou plusieurs xanthophylles, 1 à 20 mg/g d'un ou plusieurs stérols, 2 à 100 μg/g de d'un ou plusieurs phycoprostanes.

C. Composition selon le mode A ou B, caractérisée en ce qu'elle comprend 50 à 200 mg/g d'un ou plusieurs acides gras de type oméga-3, 10 à 30 mg/g d'un ou plusieurs xanthophylles, 1 à 8 mg/g d'un ou plusieurs stérols et 2 à 50 μg/g d'un ou plusieurs s phycoprostanes.

D. Composition selon l'un quelconque des modes A à C, caractérisée en ce qu'elle comprend 50 à 170 mg/g d'un ou plusieurs acides gras de type oméga-3, 10 à 25 mg/g d'un ou plusieurs xanthophylles, 1 à 6 mg/g d'un ou plusieurs stérols et 2 à 40 μg/g d'un ou plusieurs s phycoprostanes.

E. Complément alimentaire comprenant une composition selon l'un quelconque des modes A à D, et au moins une huile choisie parmi les triglycérides à chaines moyennes (MCT).

F. Complément alimentaire selon le mode E, caractérisé en ce que les triglycérides à chaines moyennes (MCT) sont choisis parmi l'huile de coco et l'huile de palme.

G. Composition selon l'un quelconque des modes A à D ou complément alimentaire selon le mode E ou F, caractérisé en ce que le ou au moins l'un des acides gras de type oméga-3 est choisi parmi l'acide stéaridonique (SDA), l'acide éicosa pentaénoïque (EPA), l'acide docosahexaénoïque (DHA) et leurs mélanges.

H. Composition selon l'un quelconque des modes A à D et G ou complément alimentaire selon l'un quelconque des modes E à G, caractérisé en ce que le ou au moins l'un des xanthophylles est la fucoxanthine.

I. Composition selon l'un quelconque des modes A à D, G et H ou complément alimentaire selon l'un quelconque des modes E à H, caractérisé en ce que le ou au moins l'un des stérols est choisi parmi les phytostérols.

J. Composition selon l'un quelconque des modes A à D et G à I ou complément alimentaire selon l'un quelconque des modes E à I, caractérisé en ce que le ou au moins l'un des phycoprostanes est choisi parmi les phytoprostanes, les isoprostanes et les neuroprostanes.

K. Composition selon l'un quelconque des modes A à D et G à J, ou complément alimentaire selon l'un quelconque des modes E à J, caractérisé en ce qu'il comprend en outre au moins un additif choisis parmi les agents conservateurs, les colorants, les arômes, les agents de délitement, les agents lubrifiants, les agents d'enrobage ou d'encapsulation.

L. Composition selon l'un quelconque des modes A à D et G à K, ou complément alimentaire selon l'un quelconque des modes E à K, caractérisé en ce qu'il se présente sous la forme de gélules, de capsules, de comprimés, de pastilles ou de poudre libre.

M. Composition selon l'un quelconque des modes A à D et G à L, ou complément alimentaire selon l'un quelconque des modes E à L, caractérisé en ce qu'il est conditionné en doses ayant un poids unitaire compris entre 10 mg et 1 g.

N. Utilisation d'une microalgue choisie dans l'une quelconque des taxons *Pinguiophyceae, Chrysophyceae, Bacillariophyceae, Mamiellophyceae, Prymnesiophyceae, Haptophyceae, Coccolithophyceae, Isochrysidaceae* et *Phaeodactylaceae,* pour préparer une composition selon l'un quelconque des modes A à D et G à M, ou un complément alimentaire selon l'un quelconque des modes E à M.

O. Utilisation selon le mode N, caractérisée en ce que ladite microalgue est la *Tisochrysis lutea* ou la *Phaeodactylum tricornutum.*

P. Utilisation d'un complément alimentaire selon l'un quelconque des modes E à M, pour prévenir l'apparition des troubles cognitifs liés au vieillissement, définis comme une diminution non pathologique des fonctions cognitives, ou les troubles cognitifs chez les enfants ou les jeunes adultes ayant subi un stress prénatal induisant des troubles non pathologiques, tels que l'hyperactivité, le déficit d'attention et de mémoire, le retard au niveau du langage et le comportement anxieux.

Q. Utilisation d'un complément alimentaire selon le mode P, pour prévenir l'apparition des troubles cognitifs liés au vieillissement, définis comme une diminution non pathologique des fonctions cognitives, caractérisée en ce que la prise journalière est comprise entre 2 à 5 mg d'extrait/kg de poids corporel.

R. Utilisation d'un complément alimentaire selon le mode P, pour prévenir l'apparition des troubles cognitifs chez les enfants ou les jeunes adultes ayant subi un stress prénatal induisant des troubles non pathologiques, tels que l'hyperactivité, le déficit d'attention et de mémoire, le retard au niveau du langage et le comportement anxieux, caractérisée en ce que la prise journalière est comprise entre 0,05 à 0,1 mg d'extrait/kg de poids corporel.

**Revendications**

1.  Complément alimentaire comprenant :

    - un extrait de *Phaeodactylum tricornutum* comprenant entre 55,1 mg/g et 78,1 mg/g d'extrait d'acides gras de type oméga-3 constitués par ALA, SDA, EPA et DHA, entre 16,0 mg/g d'extrait et 24,0 mg/g d'extrait de fucoxanthine, entre 2,4 mg/g d'extrait et 3,6 mg/g d'extrait de stérols, entre 0,002 mg/g d'extrait et 0,003 mg/g d'extrait de phycoprostanes et
    - de l'huile de coco entre 390 mg/g et 420 mg/g.

2.  Complément alimentaire selon la revendication 1, **caractérisé en ce qu'**il comprend en outre au moins un additif choisi parmi les agents conservateurs, les colorants, les arômes, les agents de délitement, les agents lubrifiants, les agents d'enrobage ou d'encapsulation.

3.  Complément alimentaire selon l'une quelconque des revendications 1 ou 2, **caractérisé en ce qu'**il se présente sous la forme de gélules, de capsules, de comprimés, de pastilles ou de poudre libre.

4.  Complément alimentaire selon l'une quelconque des revendications 1 à 3, **caractérisé en ce qu'**il **en ce qu'**il est conditionné en doses ayant un poids unitaire compris entre 10 mg et 1 g.

5.  Utilisation du complément alimentaire selon l'une quelconque des revendications 1 à 4 pour prévenir l'apparition des troubles cognitifs liés au vieillissement, définis comme une diminution non pathologique des fonctions cognitives.

6.  Utilisation selon la revendication 5, **caractérisée en ce que** la prise journalière est comprise entre 1,7 et 53 mg de complément/kg de poids corporel.

[Fig. 1]

Comportement (% alternance)

[Fig. 2]

Latence médiane

Légende:
- Sol. Saline/Véh
- D-Gal 150/Veh
- D-Gal 150/Complément HI
- D-Gal 150/Complément LOW
- D-Gal 150/Complément MED
- D-Gal 150/DHA HI

Jours

[Fig. 3]

Temps passé dans le quadrant

[Fig. 4]

Passage de latence à pas

[Fig. 5]

Taux de peroxydation lipidique

[Fig. 6]

Teneur en TNF-α (% de Veh/Veh)

[Fig. 7]

Teneur en IL-6 (% de Veh/Veh)

[Fig. 8]

Anxiété (% de locomotion dans la zone périphérique de la zone test)

[Fig. 9]

Temps d'interaction avec le même objet (%)

Fréquence d'interaction avec le même objet (%)

[Fig. 10]

Temps d'interaction (%)

Fréquence d'interaction (%)

[Fig. 11]

[Fig. 12]

[Fig. 13]

[Fig. 14]

[Fig. 15]

[Fig. 16]

[Fig. 17]

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2013032333 A1